# EUROPEAN PATENT APPLICATION

(11) **EP 4 378 948 A1**
(43) Date of publication of application: **05.06.2024**
(21) Application number: 22849616.2
(22) Date of filing: 29.07.2022
(51) Int. Cl.: C07K 4/00, A61K 38/08, A61K 51/08, A61P 13/08, A61P 35/00, C07B 59/00, C07C 273/18, C07C 275/16, C07F 5/02, G01T 1/161

(54) **RADIOLABELED COMPOUND AND USE THEREOF**

(30) Priority: 30.07.2021 JP 2021125774
(71) Applicant: OSAKA UNIVERSITY, Suita-shi Osaka 565-0871 (JP)
(72) Inventor: SHIRAKAMI, Yoshifumi, Suita-shi, Osaka 565-0871 (JP); KANEDA, Kazuko, Suita-shi, Osaka 565-0871 (JP); KADONAGA, Yuichiro, Suita-shi, Osaka 565-0871 (JP); WATABE, Tadashi, Suita-shi, Osaka 565-0871 (JP); TOYOSHIMA, Atsushi, Suita-shi, Osaka 565-0871 (JP); FUKASE, Koichi, Suita-shi, Osaka 565-0871 (JP); YOSHIYA, Taku, Ibaraki-shi, Osaka 567-0085 (JP)
(74) Representative: J A Kemp LLP
(86) International application number: PCT/JP2022/029283
(87) International publication number: WO 2023/008556

(57) **Abstract**

The aims of the present invention are to provide an agent that binds specifically to PSMA, is effective in the treatment and diagnosis of tumors or cancers expressing PSMA, for example, the treatment and diagnosis of prostate cancer, especially castration-resistant prostate cancer (CRPC), further especially metastatic castration-resistant prostate cancer (mCRPC), and does not exhibit side effects due to accumulation in the kidney or salivary glands.

The present invention provides a radiolabeled compound represented by Formula (I) or a pharmaceutically acceptable salt thereof: 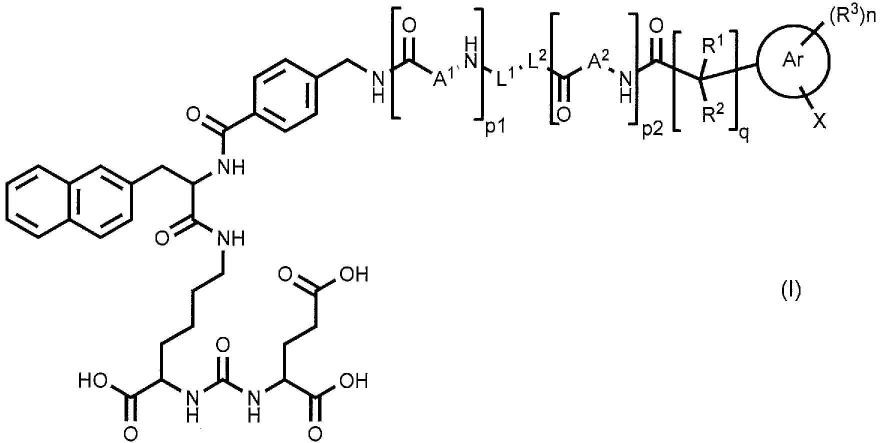 wherein each symbol is as defined in the description.

## Description

### Technical Field

The present invention relates to a radiolabeled compound useful as a therapeutic agent and/or diagnostic agent for prostate cancer, and a method for producing the same.

### Background Art

Prostate cancer is the most common cancer among men and has a good prognosis if detected early (5-year survival rate is over 95%). Initial treatment is total or partial resection of the primary tumor or radiation therapy. Hormone therapy using drugs is also an effective treatment method. However, recurrence or metastasis often occurs over time. After recurrence, hormone therapy is once successful, but it gradually becomes resistant, leading to castration-resistant prostate cancer (CRPC), especially metastasis castration-resistant prostate cancer (mCRPC), which is extremely difficult to treat. The development of effective drugs for the treatment and diagnosis of such CRPC is desired.

Prostate-specific membrane antigen (PSMA) is attracting attention as a target molecule for prostate cancer. PSMA is highly expressed not only in the primary prostate cancer, but also in recurrent lesions and in metastases in lymph nodes and bones. Therefore, it is suggested that drugs that bind specifically to PSMA may be effective in the treatment and diagnosis of CRPC and mCRPC. Since PSMA is also expressed in the kidneys and salivary glands in normal tissues, it is important that drugs that bind specifically to PSMA do not show side effects due to accumulation in the kidneys and salivary glands.

Drugs labeled with ¹⁷⁷Lu (β-ray emitting nuclide) or ²²⁵Ac (a-ray emitting nuclide) that target PSMA have been reported (Patent Document 1). Although the former drug shows a certain degree of effectiveness in treating CRPC patients, it is known that there are still cancer patients who cannot be treated with this drug. The latter drug, which is expected to have a stronger therapeutic effect, has been reported to have side effects on the salivary glands, and has also been suggested to have side effects on renal function due to progeny nuclides.

Since ²¹¹At, the same α-ray emitting nuclide as ²²⁵Ac, has a shorter half-life than ²²⁵Ac (²¹¹At: 7.2 hours, ²²⁵Ac: 10 days), drugs labeled with ²¹¹At have short action times and can be treated on an outpatient basis. Furthermore, since it is a short-lived nuclide, it also has the advantage of lower risk of prolonged side effects, and is expected to be useful as a new anticancer drug.

Various drugs labeled with ²¹¹At (a-ray emitting nuclide) that target PSMA have been reported (Patent Documents 2 and 3, Non-Patent Documents 1 and 2). However, there are problems with optimization of pharmacokinetics and side effects due to kidney accumulation, and no drug has progressed to the clinical trial stage at this time.

Drugs labeled with ¹⁸F that target PSMA have been reported to be useful as PET imaging diagnostic agents (Patent Document 4) .

### Document List

### Patent Document

[Patent Document 1] WO 2015/055318
[Patent Document 2] WO 2010/014933A2
[Patent Document 3] WO 2017/070482A2
[Patent Document 4] EP3805250A1

### Non-Patent Document

[Non-Patent Document 1] J Nucl Med 2016; 57:1569-75
[Non-Patent Document 2] Nucl Med Biol 2021; 94-95:67-80

### Summary of the Invention

### Problems to be Solved by the Invention

The aims of the present invention are to provide an agent that binds specifically to PSMA, is effective in the treatment and diagnosis of tumors or cancers expressing PSMA, for example, the treatment and diagnosis of prostate cancer, especially castration-resistant prostate cancer (CRPC), further especially metastatic castration-resistant prostate cancer (mCRPC), and does not exhibit side effects due to accumulation in the kidney or salivary glands.

### Means of Solving the Problems

The present inventors have conducted intensive studies in an attempt to solve the above-mentioned problems and found that a novel radiolabeled compound represented by the following Formula (I) binds specifically to PSMA, is effective in the treatment and diagnosis of tumors or cancers expressing PSMA, for example, the treatment and diagnosis of prostate cancer, especially castration-resistant prostate cancer (CRPC), further especially metastatic castration-resistant prostate cancer (mCRPC), and does not exhibit side effects due to accumulation in the kidney or salivary glands, which resulted in the completion of the present invention.

Accordingly, the present invention provides the following.
[1] a radiolabeled compound represented by Formula (I) or a pharmaceutically acceptable salt thereof (hereinafter to be also referred to as Radiolabeled Compound (I)): 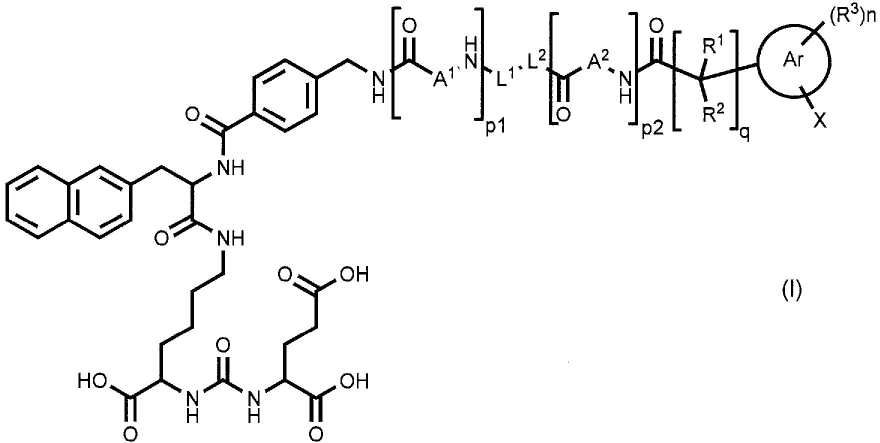 wherein
   -CO-A¹-NH- in the number of p1 are each independently an amino acid residue;
   -CO-A²-NH- in the number of p2 are each independently an amino acid residue;
   L¹ is a single bond, or -CO-(CH₂)ₘ₁-CO- wherein m1 is an integer of 1 to 6;
   L² is a single bond, or -NH-(CH₂)ₘ₂-CH(COOH)-NH- wherein m2 is an integer of 1 to 6;
   Ar is a C₆₋₁₄ aryl group;
   R¹ in the number of q are each independently a hydrogen atom, a C₁₋₆ alkyl group or an amino group;
   R² in the number of q are each independently a hydrogen atom or a C₁₋₆ alkyl group;
   R³ in the number of n are each independently a C₁₋₆ alkyl group or a hydroxy group;
   X is a radionuclide selected from ²¹¹At, ²¹⁰At, ¹³¹I, ¹²⁵I, ¹²⁴I, ¹²³I, ⁷⁷Br and ⁷⁶Br;
   p1 is an integer of 0 to 3;
   p2 is an integer of 0 to 3;
   q is an integer of 0 to 3; and
   n is an integer of 0 to 3.
[2] The compound according to the above-mentioned [1] or a pharmaceutically acceptable salt thereof, wherein L¹ and L² are both single bonds.
[3] The compound according to the above-mentioned [1] or a pharmaceutically acceptable salt thereof, wherein L¹ is -CO-(CH₂)ₘ₁-CO- wherein m1 is as defined in the above-mentioned [1], and L² is -NH-(CH₂)ₘ₂-CH(COOH)-NH- wherein m2 is as defined in the above-mentioned [1].
[4] The compound according to any one of the above-mentioned [1] to [3] or a pharmaceutically acceptable salt thereof, wherein at least one of -CO-A¹-NH- in the number of p1 is a glutamic acid residue.
[5] The compound according to any one of the above-mentioned [1] to [4] or a pharmaceutically acceptable salt thereof, wherein p1 is an integer of 0 to 2.
[6] The compound according to any one of the above-mentioned [1] to [5] or a pharmaceutically acceptable salt thereof, wherein at least one of -CO-A²-NH- in the number of p2 is a glycine acid residue.
[7] The compound according to any one of the above-mentioned [1] to [6] or a pharmaceutically acceptable salt thereof, wherein p2 is 0 or 1.
[8] The compound according to any one of the above-mentioned [1] to [7] or a pharmaceutically acceptable salt thereof, wherein Ar is a phenyl group.
[9] The compound according to any one of the above-mentioned [1] to [8] or a pharmaceutically acceptable salt thereof, wherein R¹ and R² are hydrogen atoms.
[10] The compound according to any one of the above-mentioned [1] to [9] or a pharmaceutically acceptable salt thereof, wherein q is an integer of 1 to 3.
[11] A pharmaceutical composition comprising a compound as defined in any one of the above-mentioned [1] to [10] or a pharmaceutically acceptable salt thereof, and pharmaceutically acceptable carrier.
[12] A therapeutic agent for a tumor or cancer expressing prostate-specific membrane antigen (PSMA)), comprising a compound as defined in any one of the above-mentioned [1] to [10] or a pharmaceutically acceptable salt thereof.
[13] The agent according to above-mentioned [12], wherein the tumor or cancer expressing PSMA is prostate cancer.
[14] A diagnostic agent for a tumor or cancer expressing prostate-specific membrane antigen (PSMA)), comprising a compound as defined in any one of the above-mentioned [1] to [10] or a pharmaceutically acceptable salt thereof.
[15] The agent according to above-mentioned [14], wherein the tumor or cancer expressing PSMA is prostate cancer.
[16] A compound represented by Formula (II) or a salt thereof (hereinafter to be also referred to as Boronic Acid Compound (II)) : 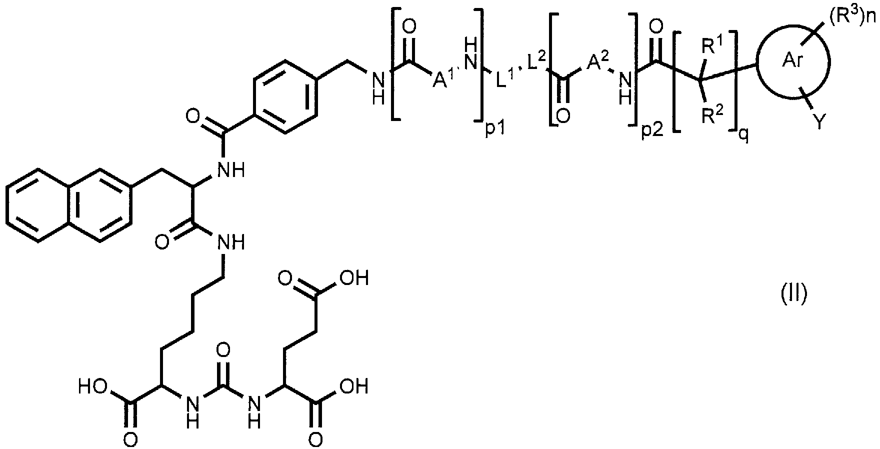 wherein
   -CO-A¹-NH- in the number of p1 are each independently an amino acid residue;
   -CO-A²-NH- in the number of p2 are each independently an amino acid residue;
   L¹ is a single bond, or -CO-(CH₂)ₘ₁-CO- wherein m1 is an integer of 1 to 6;
   L² is a single bond, or -NH-(CH₂)ₘ₂-CH(COOH)-NH- wherein m2 is an integer of 1 to 6;
   Ar is a C₆₋₁₄ aryl group;
   R¹ in the number of q are each independently a hydrogen atom, a C₁₋₆ alkyl group or an amino group;
   R² in the number of q are each independently a hydrogen atom or a C₁₋₆ alkyl group;
   R³ in the number of n are each independently a C₁₋₆ alkyl group or a hydroxy group;
   Y is a boryl group (-B(OH)₂) or its ester group;
   p1 is an integer of 0 to 3;
   p2 is an integer of 0 to 3;
   q is an integer of 0 to 3; and
   n is an integer of 0 to 3.
[17] The compound according to the above-mentioned [16] or a salt thereof, wherein Y is a boryl group (-B(OH)₂) or a 4,4,5,5-tetramethyl-1,3,2-dioxaboran-2-yl group.
[18] A method for producing a radiolabeled compound represented by Formula (I) or a pharmaceutically acceptable salt thereof, comprising the following step; 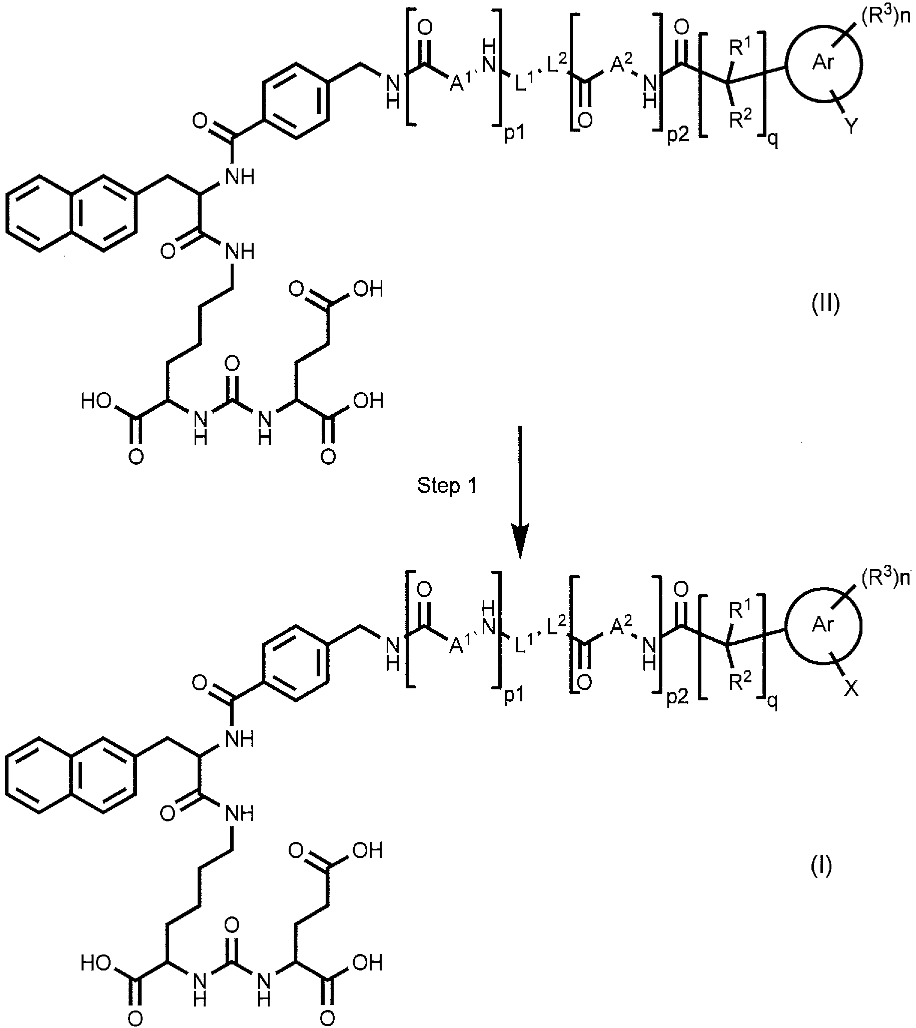 wherein
   -CO-A¹-NH- in the number of p1 are each independently an amino acid residue;
   -CO-A²-NH- in the number of p2 are each independently an amino acid residue;
   L¹ is a single bond, or -CO-(CH₂)ₘ₁-CO- wherein m1 is an integer of 1 to 6;
   L² is a single bond, or -NH-(CH₂)ₘ₂-CH(COOH)-NH- wherein m2 is an integer of 1 to 6;
   Ar is a C₆₋₁₄ aryl group;
   R¹ in the number of q are each independently a hydrogen atom, a C₁₋₆ alkyl group or an amino group;
   R² in the number of q are each independently a hydrogen atom or a C₁₋₆ alkyl group;
   R³ in the number of n are each independently a C₁₋₆ alkyl group or a hydroxy group;
   X is a radionuclide selected from ²¹¹At, ²¹⁰At, ¹³¹I, ¹²⁵I, ¹²⁴I, ¹²³I, ⁷⁷Br and ⁷⁶Br;
   Y is a boryl group (-B(OH)z) or its ester group;
   p1 is an integer of 0 to 3;
   p2 is an integer of 0 to 3;
   q is an integer of 0 to 3; and
   n is an integer of 0 to 3,
   Step 1: a step of reacting a compound represented by Formula (II) or a salt thereof with a radionuclide selected from ²¹¹At, ²¹⁰At, ¹³¹I, ¹²⁵I, ¹²⁴I, ¹²³I, ⁷⁷Br and ⁷⁶Br in the presence of a reagent selected from an alkali metal iodide, an alkali metal bromide, N-bromosuccinimide, N-chlorosuccinimide, N-iodosuccinimide and hydrogen peroxide, in water to obtain a radiolabeled compound represented by Formula (I) or a pharmaceutically acceptable salt thereof.

### Effect of the Invention

According to the present invention, a radiolabeled compound that binds specifically to PSMA, is effective in the treatment and diagnosis of tumors or cancers expressing PSMA, for example, the treatment and diagnosis of prostate cancer, especially castration-resistant prostate cancer (CRPC), further especially metastatic castration-resistant prostate cancer (mCRPC), and does not exhibit side effects due to accumulation in the kidney or salivary glands, can be provided.

### Brief Description of the Drawings

[Figure 1]

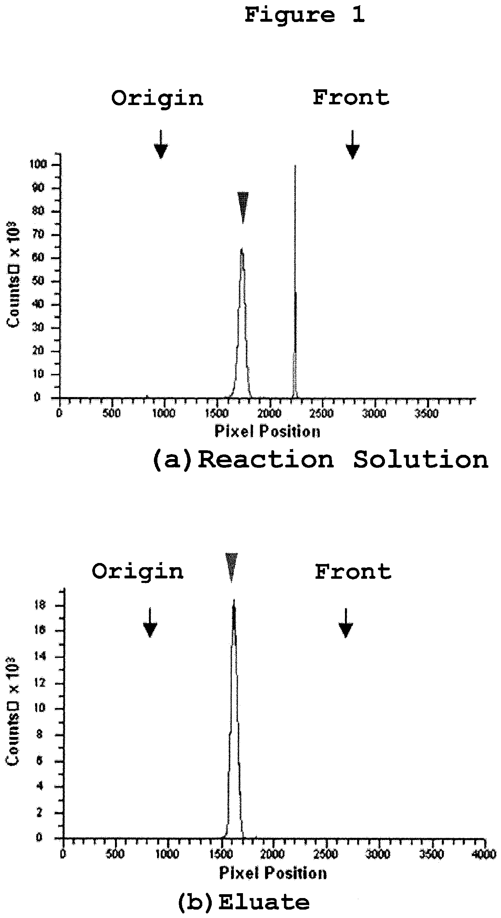

   Figure 1 shows the analysis results of the reaction solution and eluate obtained in Example 3 by thin-layer chromatograph method (TLC). Figure 1(a) shows the analysis results of the reaction solution, and Figure 1(b) shows the analysis results of the eluate.
[Figure 2]

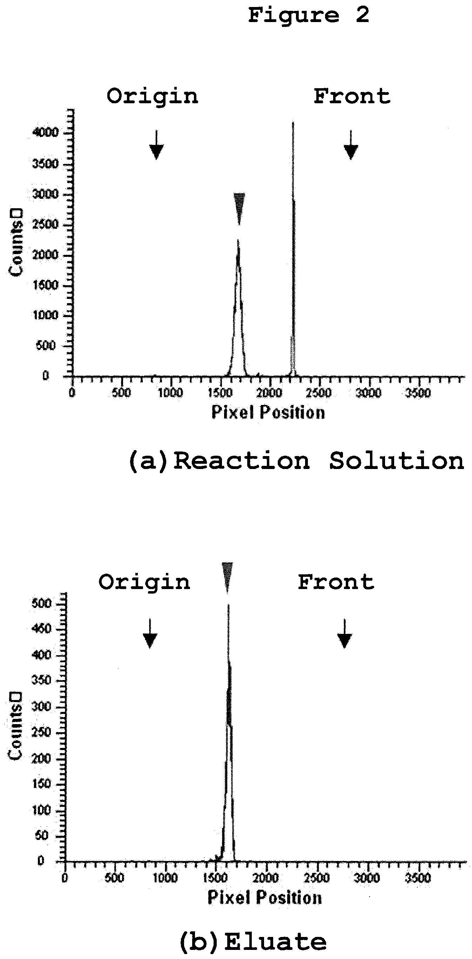

   Figure 2 shows the analysis results of the reaction solution and eluate obtained in Example 4 by thin-layer chromatograph method (TLC). Figure 2(a) shows the analysis results of the reaction solution, and Figure 2(b) shows the analysis results of the eluate.
[Figure 3]

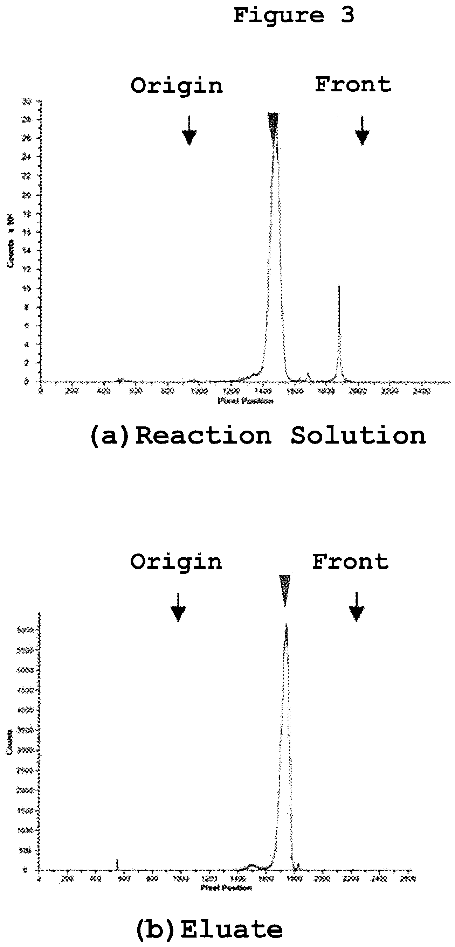

   Figure 3 shows the analysis results of the reaction solution and eluate obtained in Example 7 by thin-layer chromatograph method (TLC). Figure 3(a) shows the analysis results of the reaction solution, and Figure 3(b) shows the analysis results of the eluate.
[Figure 4]

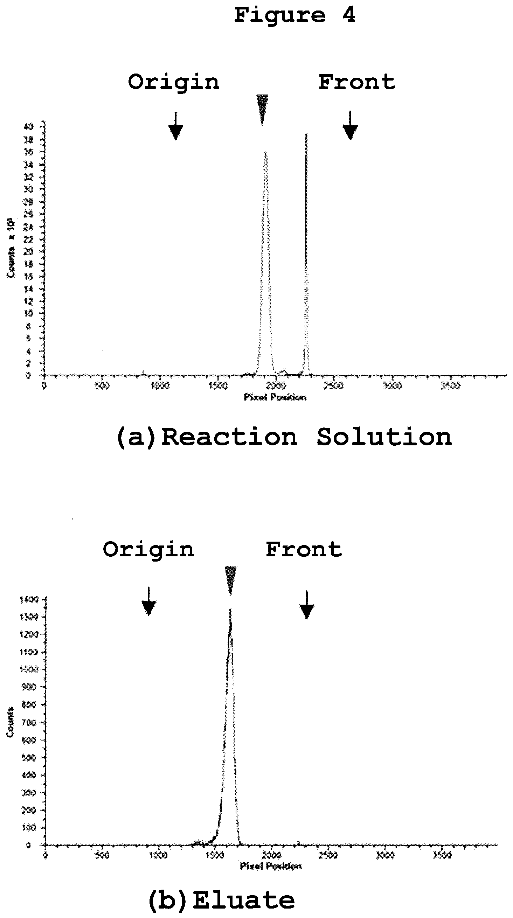

   Figure 4 shows the analysis results of the reaction solution and eluate obtained in Example 8 by thin-layer chromatograph method (TLC). Figure 4(a) shows the analysis results of the reaction solution, and Figure 4(b) shows the analysis results of the eluate.
[Figure 5]

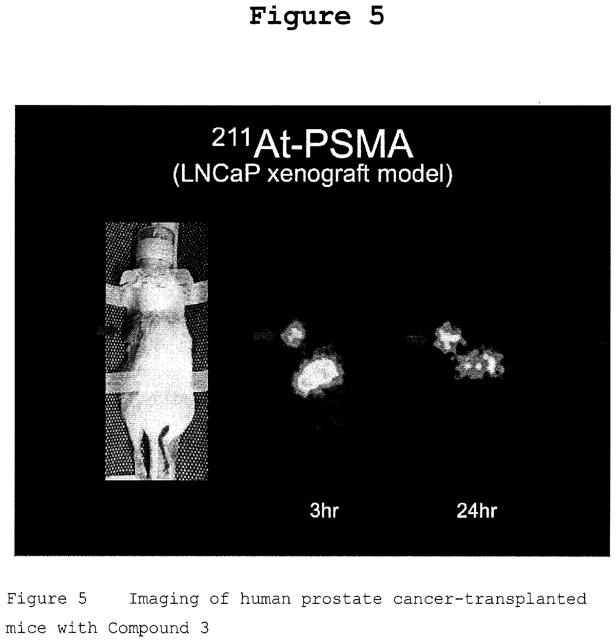

   Figure 5 shows the imaging of human prostate cancer-transplanted mice with Compound 3 in Experimental Example 1.
[Figure 6]

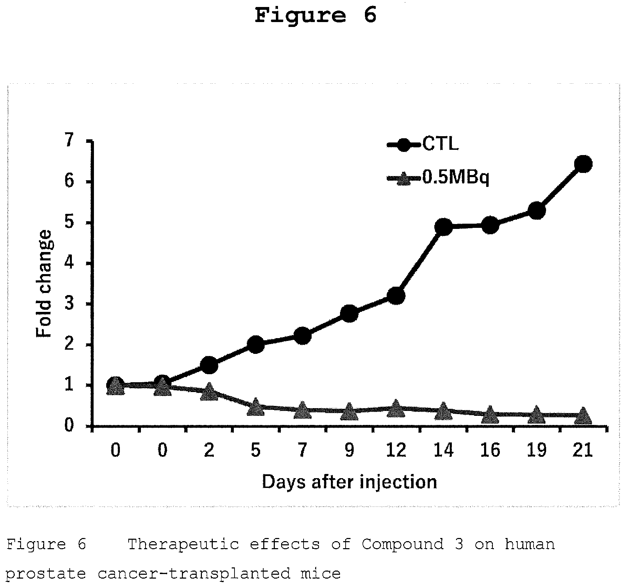

   Figure 6 shows the therapeutic effects of Compound 3 on human prostate cancer-transplanted mice in Experimental Example 2.
[Figure 7]

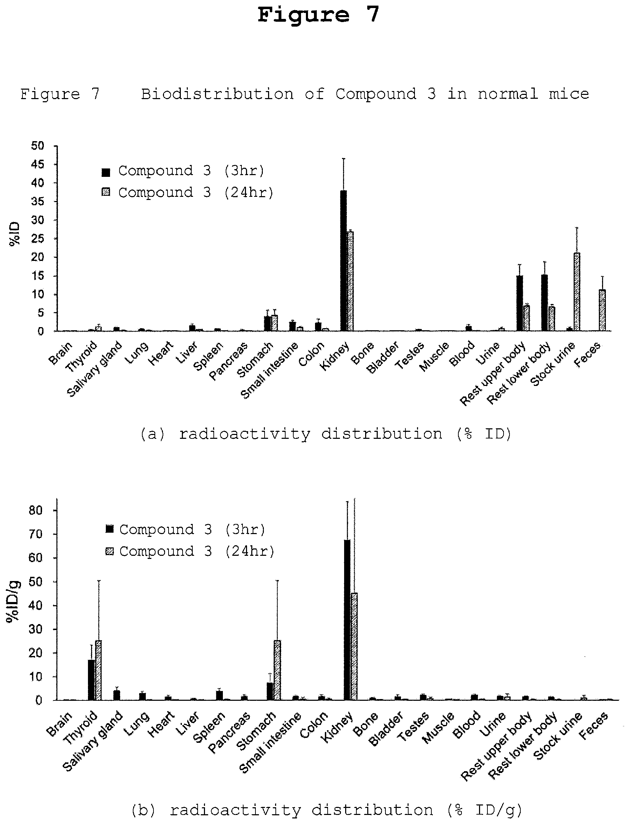

   Figure 7 shows the biodistribution of Compound 3 in normal mice in Experimental Example 3. Figure 7(a) shows radioactivity distribution, and Figure 7(b) shows radioactivity distribution per 1 g of organ weight.
[Figure 8]

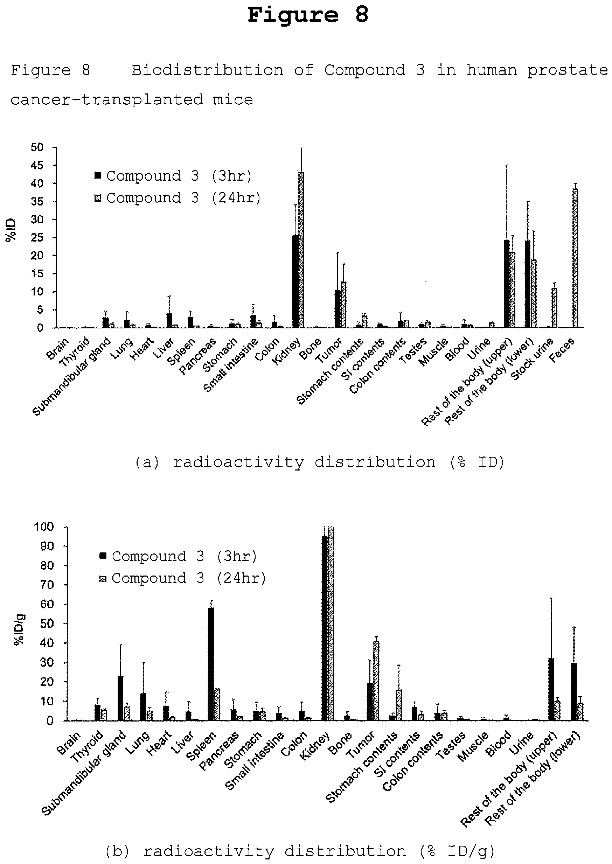

   Figure 8 shows the biodistribution of Compound 3 in human prostate cancer-transplanted mice in Experimental Example 3. Figure 8(a) shows radioactivity distribution, and Figure 8(b) shows radioactivity distribution per 1 g of organ weight.
[Figure 9]

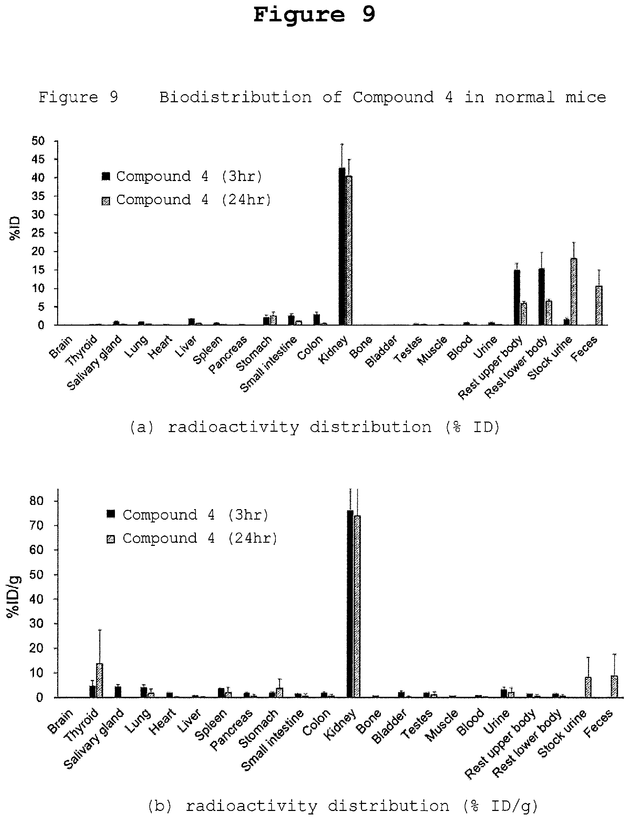

   Figure 9 shows the biodistribution of Compound 4 in normal mice in Experimental Example 3. Figure 9(a) shows radioactivity distribution, and Figure 9(b) shows radioactivity distribution per 1 g of organ weight.
[Figure 10]

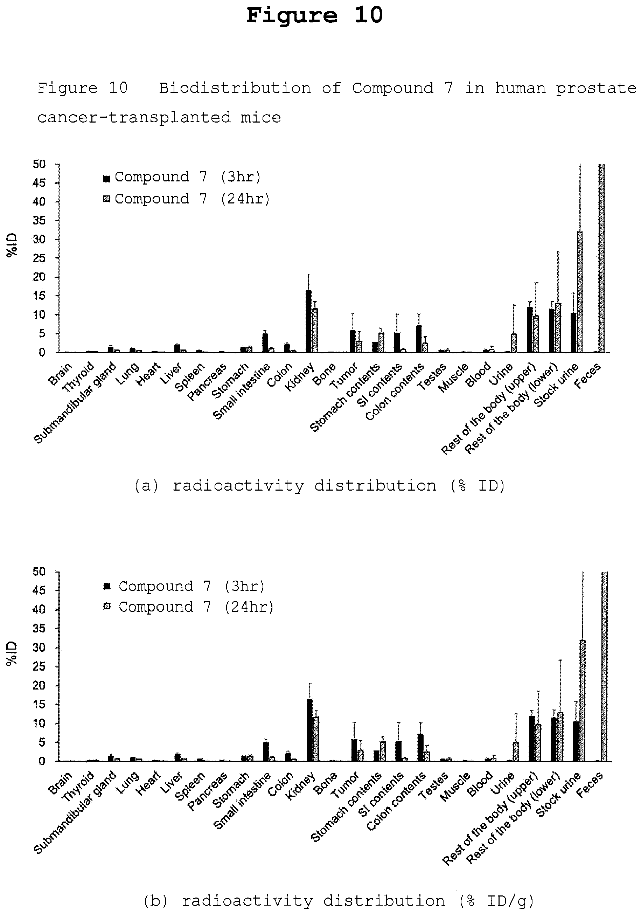

   Figure 10 shows the biodistribution of Compound 7 in human prostate cancer-transplanted mice in Experimental Example 3. Figure 10(a) shows radioactivity distribution, and Figure 10(b) shows radioactivity distribution per 1 g of organ weight.
[Figure 11]

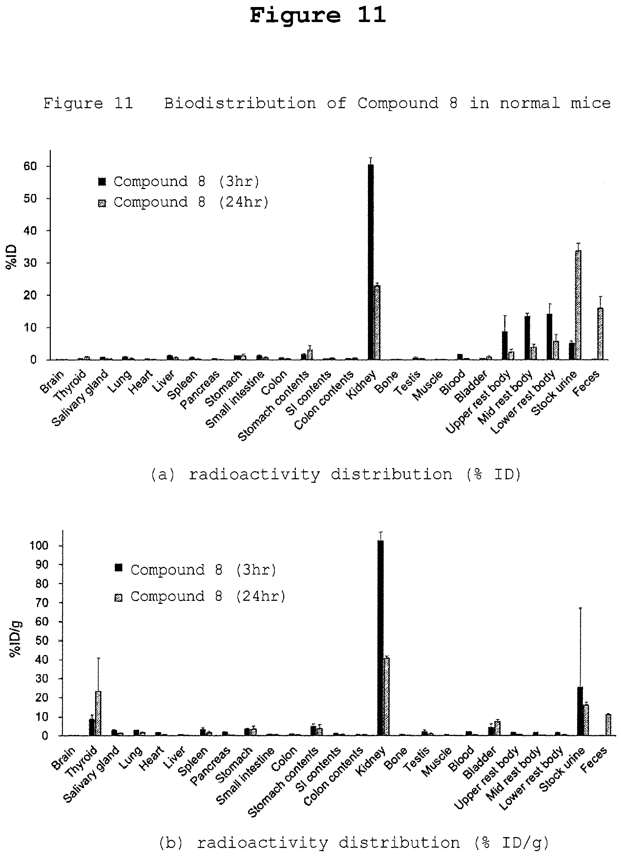

   Figure 11 shows the biodistribution of Compound 8 in normal mice in Experimental Example 3. Figure 11(a) shows radioactivity distribution, and Figure 11(b) shows radioactivity distribution per 1 g of organ weight.
[Figure 12]

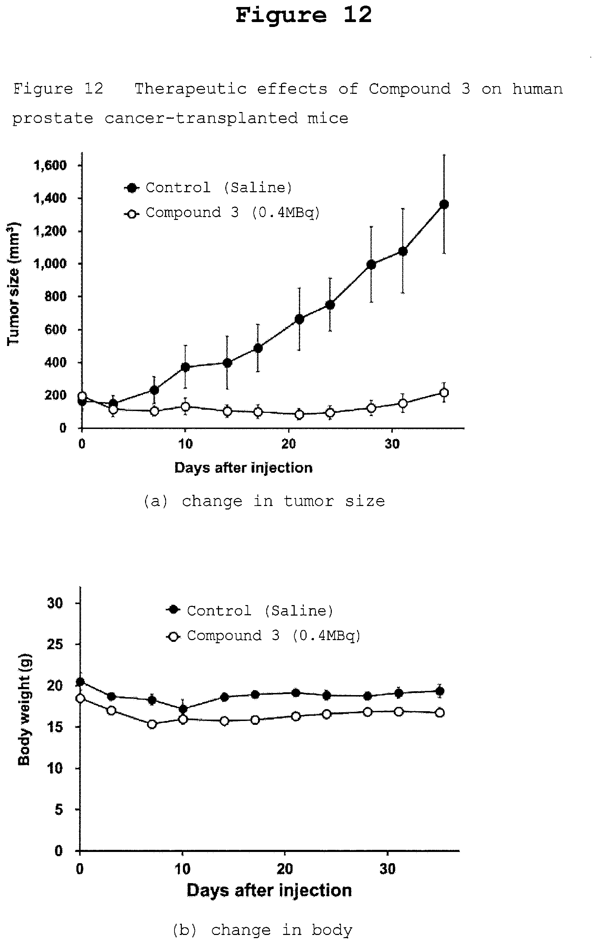

   Figure 12 shows the therapeutic effects of Compound 3 on human prostate cancer-transplanted mice in Experimental Example 4. Figure 12(a) shows a change in tumor size, and Figure 12(b) shows a change in body weight.
[Figure 13]

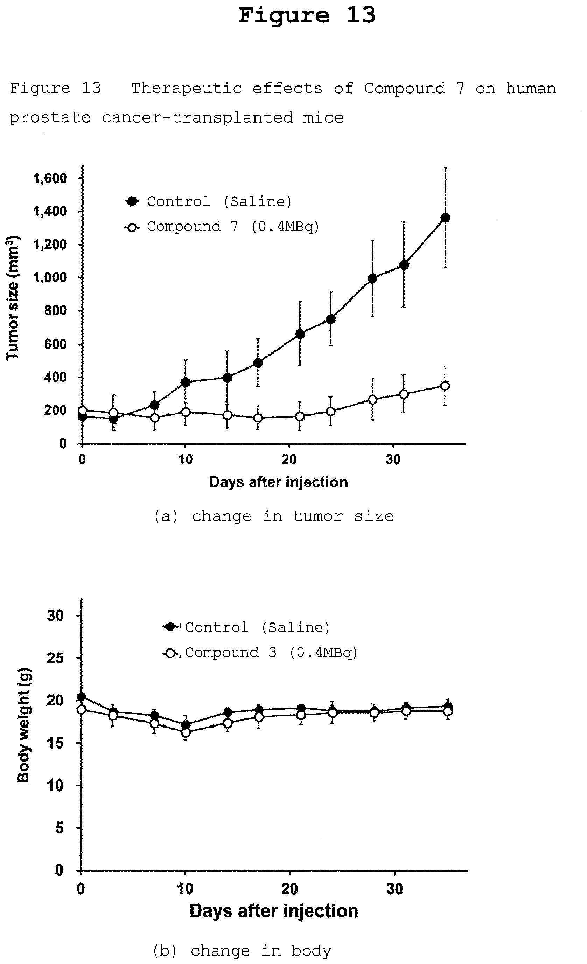

   Figure 13 shows the therapeutic effects of Compound 7 on human prostate cancer-transplanted mice in Experimental Example 4. Figure 13(a) shows a change in tumor size, and Figure 13(b) shows a change in body weight.
[Figure 14]

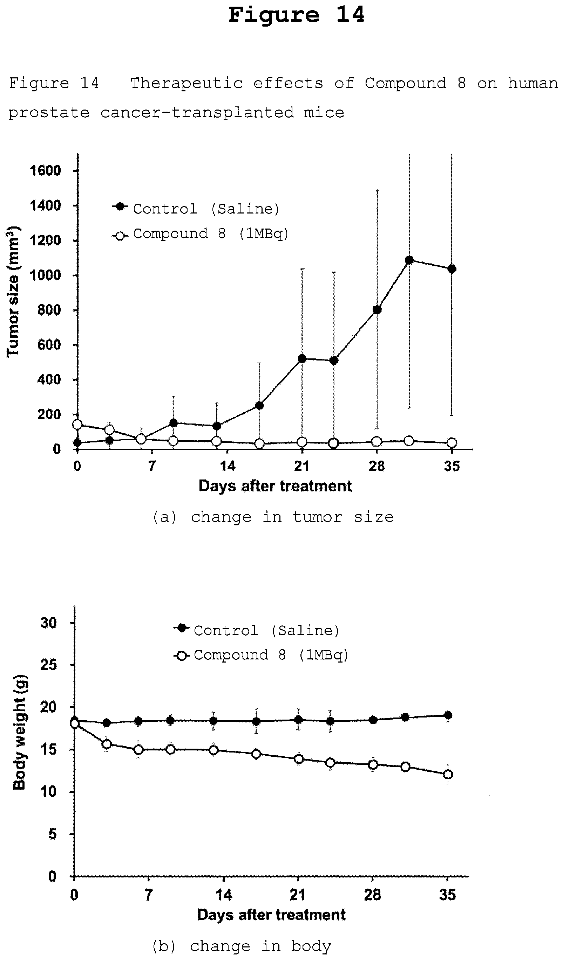

   Figure 14 shows the therapeutic effects of Compound 8 on human prostate cancer-transplanted mice in Experimental Example 4. Figure 14(a) shows a change in tumor size, and Figure 14(b) shows a change in body weight.

### Description of Embodiments

The present invention is explained in detail in the following.

In the present specification, examples of the "C₁₋₃ alkyl group" include methyl, ethyl, propyl and isopropyl.

In the present specification, examples of the "C₁₋₆ alkyl group" include methyl, ethyl, propyl, isopropyl, butyl, isobutyl, sec-butyl, tert-butyl, pentyl, isopentyl, neo-pentyl, 1-ethylpropyl, hexyl, isohexyl, 1,1-dimethylbutyl, 2,2-dimethylbutyl, 3,3-dimethylbutyl and 2-ethylbutyl. The preferred is "C₁₋₃ alkyl group".

In the present specification, examples of the "C₆₋₁₄ aryl group" include phenyl, 1-naphthyl, 2-naphthyl, 1-anthryl, 2-anthryl and 9-anthryl.

In the present specification, the "amino acid residue" means a divalent group obtained by removing H from the amino group and OH from the carboxy group of an amino acid. The amino acid of the amino acid residue is not particularly limited as long as it has an amino group and a carboxy group, and it may be a natural type (L-type) or an unnatural type (D-type) amino acid, or may be an artificial amino acid. Further, the amino acid may be an α-amino acid, a β-amino acid, a γ-amino acid or the like. It may be a cyclic amino acid as shown below. 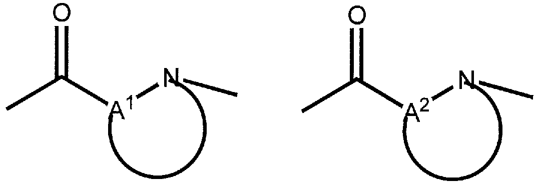 wherein each symbol in the formulas is as defined above.

Examples of the α-amino acid include glycine, alanine, valine, leucine, isoleucine, serine, threonine, cysteine, methionine, glutamic acid, aspartic acid, lysine, arginine, histidine, glutamine, asparagine, phenylalanine, tyrosine, α-methyltyrosine, tryptophan, ornithine, thyroxine, proline, 3,4-dihydroxyphenylalanine, 3-(1-naphthyl)alanine, 3-(2-naphthyl)alanine, α-aminobutyric acid, norvaline, norleucine, homonorleucine, 1,2,4-triazole-3-alanine, 2-aminoadipic acid, propargylglycine, allylglycine, α-cyclobutylmethylglycine, 6-azidonorleucine, 4-azidophenylalanine, 4-fluoroglutamic acid, 4-iodophenylalanine and the like;
Examples of the β-amino acid include β-alanine, 3-aminoadipic acid and the like;
Examples of the γ-amino acid include γ-aminobutyric acid and the like. When the amino acid has a functional group in its side chain, the functional group may be protected/modified.

In the present specification, the "boryl group (-B(OH)₂)" is also referred to as a dihydroxyboryl group.

In the present specification, examples of the "ester group of a boryl group" include the following groups. 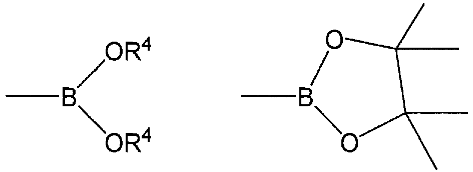 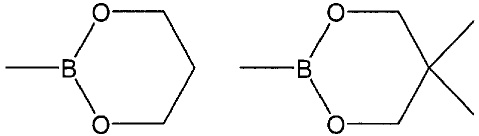 wherein R⁴ is a C₁₋₆ alkyl group.

In the present specification, the "protected amino acid residue" means an amino acid residue whose functional group is protected when the amino acid residue has a functional group. When it has an amino group, it is protected with an amino-protecting group such as a tert-butoxycarbonyl group (Boc group), and when it has a carboxyl group, it is protected with a carboxy-protecting group such as a tert-butyl group. These protecting groups are appropriately selected depending on the type of other protecting groups and resin for solid phase synthesis, the synthesis strategy, and the like.

In the present specification, the "protected hydroxy group" is a hydroxy group protected by a "hydroxy-protecting group", and examples of the "hydroxy-protecting group" include a benzyl group, a p-methoxybenzyl group, a methoxymethyl group, a trimethylsilyl group, a triethylsilyl group, a trityl group, a tert-butyl group, a tert-butyldimethylsilyl group, a tetrahydropyranyl group and the like.

In the present specification, the "protected amino group" is an amino group protected by an "amino-protecting group", and examples of the "amino-protecting group" include a 9-fluorenylmethyloxycarbonyl group (Fmoc group), Boc group, a benzyloxycarbonyl group (Cbz group) and the like.

In the present specification, examples of the "carboxy-protecting group" include a tert-butyl group, a benzyl group, a C₁₋₂ alkyl group (a methyl group, an ethyl group), diphenylmethyl group and the like.

Radiolabeled Compound (I) of the present invention is the compound shown below. 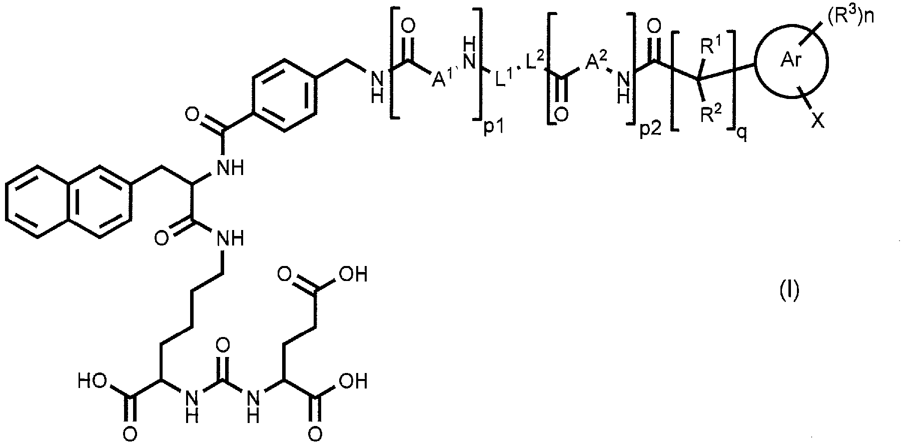 wherein each symbol in the formula is as defined above.
-CO-A¹-NH- in the number of p1 are each independently an amino acid residue.

In one embodiment, the amino acid residue is preferably a glutamic acid residue (Glu).

In one embodiment, at least one of the amino acid residues in the number of p1 is preferably a glutamic acid residue.

In another embodiment, at least two of the amino acid residues in the number of p1 are preferably glutamic acid residues.

The configuration of the amino acid residue is not particularly limited, and may be any of D-form, L-form and DL-form (that is, any of R-form, S-form and R/S-form).

p1 is an integer of 0 to 3.

In one embodiment, p1 is preferably an integer of 0 to 2.

In one embodiment, -(CO-A¹-NH)p1- is preferably a single bond, or -L-Glu-L-Glu- or -D-Glu-D-Glu-.

-CO-A²-NH- in the number of p2 are each independently an amino acid residue.

In one embodiment, the amino acid residue is preferably a glycine residue (Gly).

In one embodiment, at least one of the amino acid residues in the number of p2 is preferably a glycine acid residue.

The configuration of the amino acid residue is not particularly limited, and may be any of D-form, L-form and DL-form (that is, any of R-form, S-form and R/S-form).

p2 is an integer of 0 to 3.

In one embodiment, p2 is preferably 0 or 1.

In one embodiment, -(CO-A²-NH)p2- is preferably a single bond, or -Gly-.

L¹ is a single bond, or -CO-(CH₂)ₘ₁-CO- wherein m1 is an integer of 1 to 6.

In one embodiment, m1 is preferably 2 or 3, particularly preferably 2.

L² is a single bond, or -NH-(CH₂)ₘ₂-CH(COOH)-NH- wherein m2 is an integer of 1 to 6.

In one embodiment, m2 is preferably an integer of 3 to 5, particularly preferably 4.

The preferred combinations of L¹ and L² include
(1) a combination in which L¹ and L² are both single bonds, and
(2) a combination in which L¹ is -CO-(CH₂)ₘ₁-CO- wherein m1 is as defined above, and L² is -NH-(CH₂)ₘ₂-CH(COOH)-NH- wherein m2 is as defined above.

R¹ in the number of q are each independently a hydrogen atom, a C₁₋₆ alkyl group (e.g., methyl) or an amino group.

In one embodiment, R¹ is preferably a hydrogen atom.

R² in the number of q are each independently a hydrogen atom or a C₁₋₆ alkyl group (e.g., methyl).

In one embodiment, R² is preferably a hydrogen atom.

q is an integer of 0 to 3.

In one embodiment, q is preferably an integer of 1 to 3, more preferably 1.

Ar is a C₆₋₁₄ aryl group.

In one embodiment, Ar is preferably a phenyl group.

R³ in the number of n are each independently a C₁₋₆ alkyl group (e.g., methyl) or a hydroxy group.

n is an integer of 0 to 3.

In one embodiment, n is preferably 0.

X is a radionuclide selected from ²¹¹At (α-ray emitting nuclide), ²¹⁰At (a-ray emitting nuclide), ¹³¹I (β-ray emitting nuclide), ¹²⁵I (X-ray emitting nuclide), ¹²⁴I (positron emitting nuclide), ¹²³I (γ-ray emitting nuclide), ⁷⁷Br (auger electron emitting nuclide) and ⁷⁶Br (positron emitting nuclide).

The half-lives of these radionuclides are 7.2 hours for ²¹¹At, 8.3 hours for ²¹⁰At, 8.04 days for ¹³¹I, 59.4 days for ¹²⁵I, 4.2 days for ¹²⁴I, 13.2 hours for ¹²³I, 57 hours for ⁷⁷Br, and 16 hours for ⁷⁶Br.

The bonding position of X on Ar is not particularly limited. For example, when Ar is a phenyl group, the bonding position of X is preferably the 3-position or the 4-position.

The configuration of the naphthyl alanine residue in Formula (I) 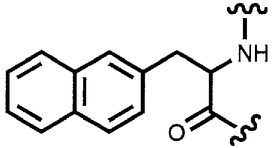 is not particularly limited, and may be any of D-form, L-form and DL-form (that is, any of R-form, S-form and R/S-form).

The configuration of the lysine-glutamic acid residue in Formula (I) 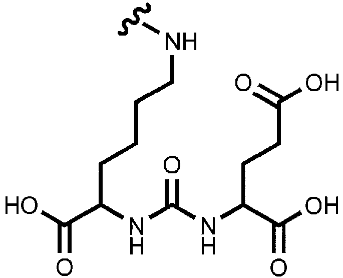 is not particularly limited, and may be any of the following configurations.

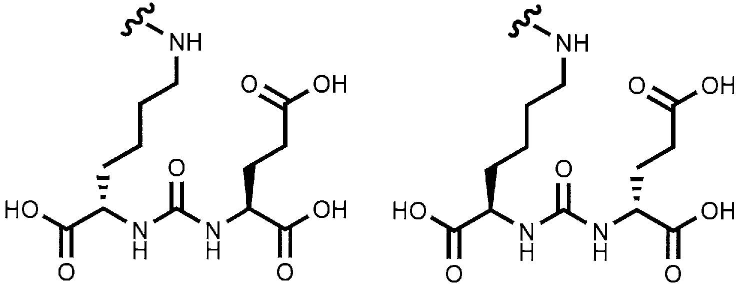

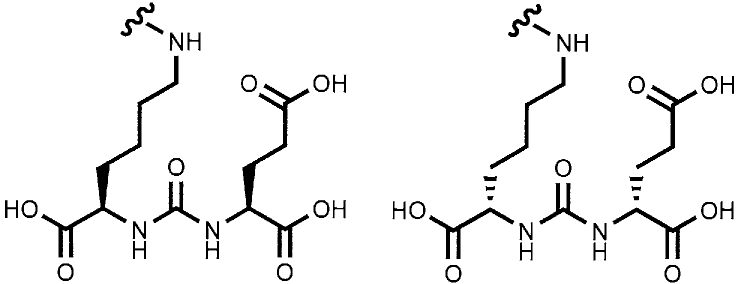

In one embodiment, the lysine-glutamic acid residue is preferably

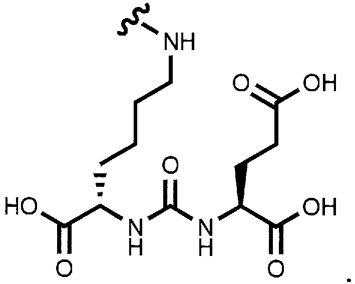

That is, Compound (I) is preferably Compound (Ia) shown below. 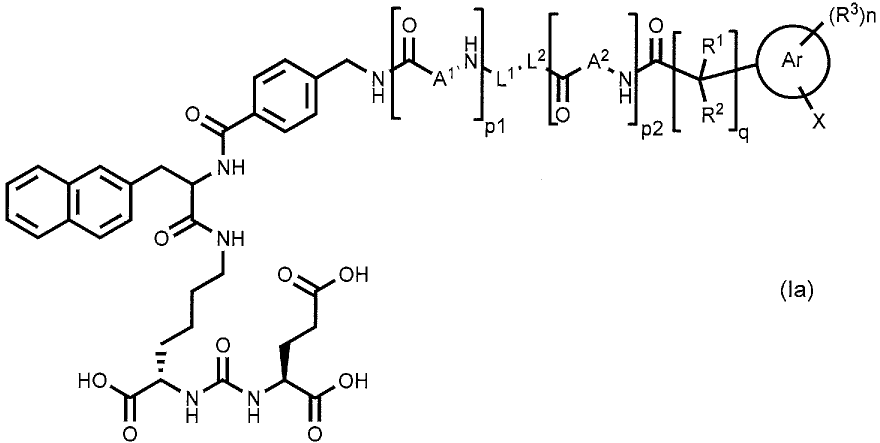 wherein each symbol in the formula is as defined above.

Specific examples of Radiolabeled Compound (I) include the followings. 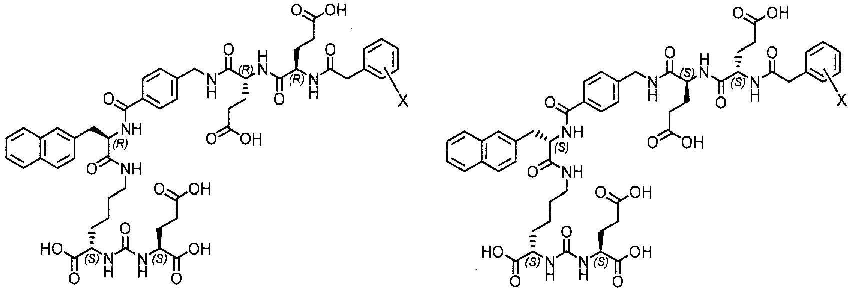 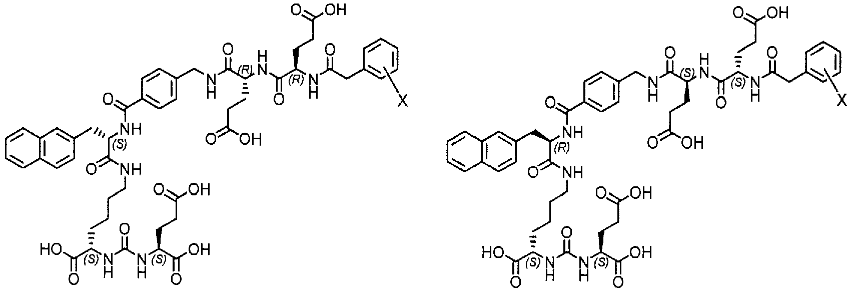 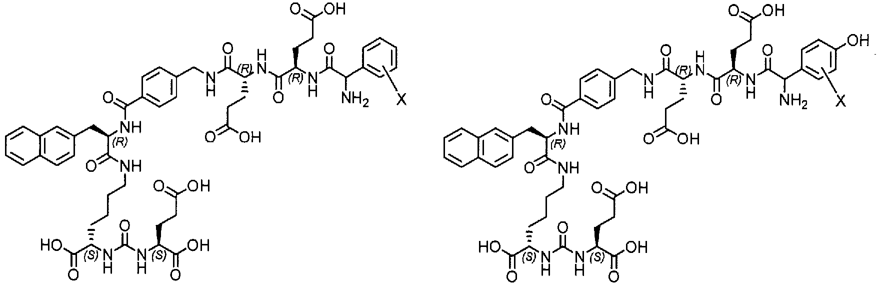 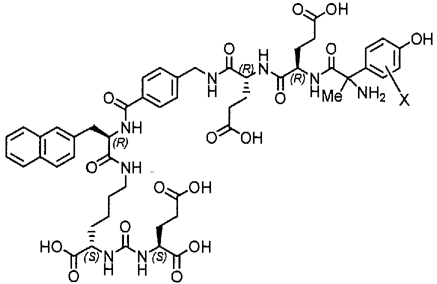 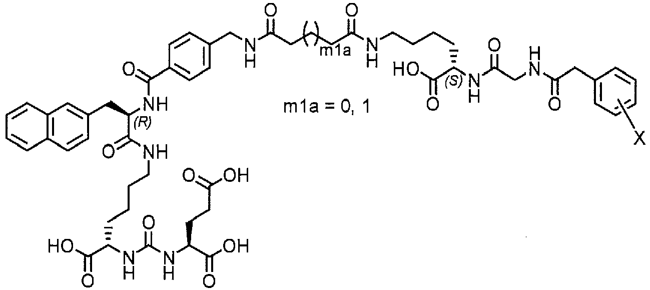 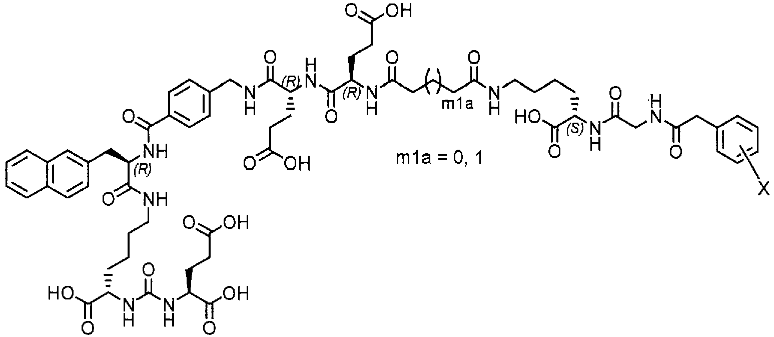 wherein X is as defined above.

A compound represented by Formula (I) may be in the form of a pharmaceutically acceptable salt thereof. As the pharmaceutically acceptable salt, for example, when the compound has an acidic functional group, examples of the salt include inorganic salts such as alkali metal salts (e.g., sodium salt, potassium salt, etc.), alkaline-earth metal salts (e.g., calcium salt, magnesium salt, barium salt, etc.) and ammonium salt, and when the compound has a basic functional group, examples of the salt include salts with inorganic acids such as hydrogen chloride, hydrobromic acid, nitric acid, sulfuric acid and phosphoric acid, and salts with organic acids such as acetic acid, phthalic acid, fumaric acid, oxalic acid, tartaric acid, maleic acid, citric acid, succinic acid, methanesulfonic acid and p-toluenesulfonic acid.

The chiral C atom in Radiolabeled Compound (I) may be in D-configuration or in L-configuration. Radiolabeled Compound (I) has optical isomers based on the chiral C atom, and any optical isomer and mixture thereof in any proportion are also included in Radiolabeled Compound (I).

A method for producing Radiolabeled Compound (I) of the present invention will be explained below.

In the present specification, when a raw material compound is in the form of a salt, examples of such salt include metal salts (e.g., alkali metal salts such as sodium salt and potassium salt; and alkaline-earth metal salts such as calcium salt, magnesium salt and barium salt), ammonium salts, salts with organic bases (e.g., trimethylamine, triethylamine, pyridine, picoline, 2,6-lutidine), salts with inorganic acids (e.g., hydrochloric acid, hydrobromic acid, nitric acid, sulfuric acid), salts with organic acids (e.g., formic acid, acetic acid, trifluoroacetic acid, phthalic acid, fumaric acid, oxalic acid, tartaric acid, maleic acid, citric acid, succinic acid, malic acid), and the like.

Radiolabeled Compound (I) can be produced by the following method comprising Step 1. 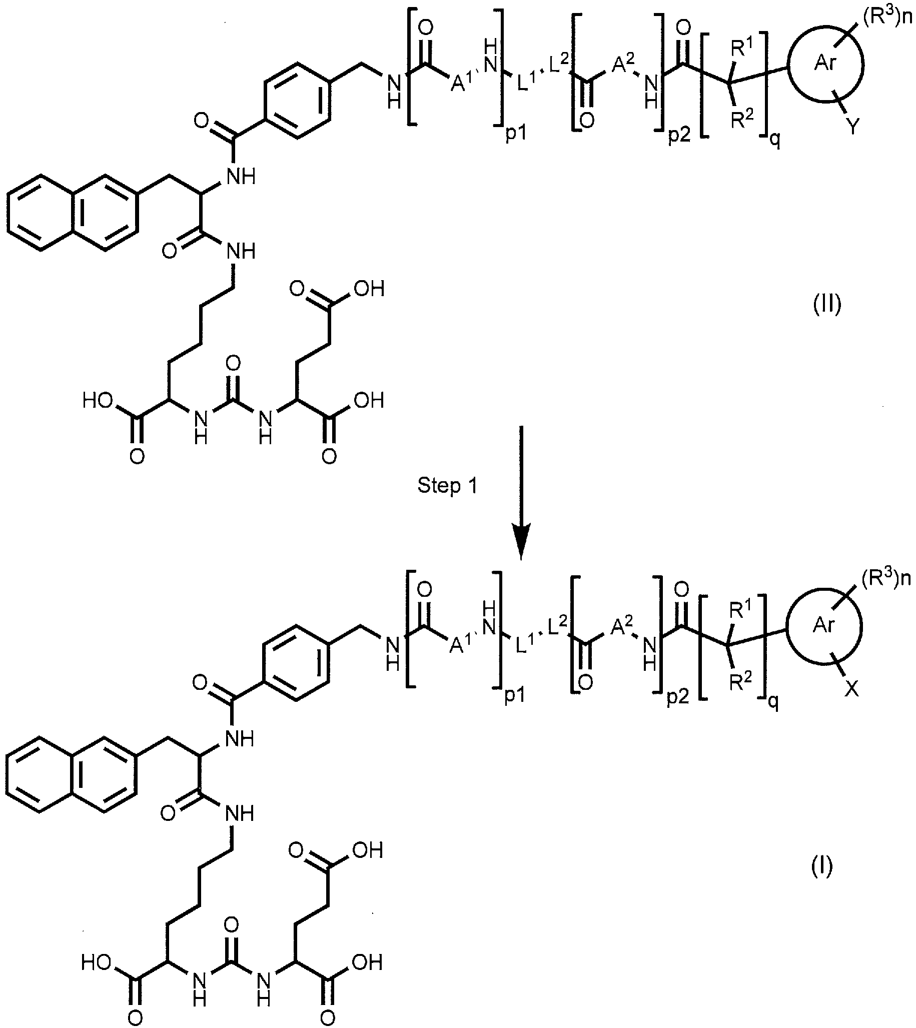 wherein each symbol in the formula is as defined above.

Y is a boryl group (-B(OH)₂) or its ester group.

Y is preferably a boryl group (-B(OH)₂) or a 4,4,5,5-tetramethyl-1,3,2-dioxaboran-2-yl group (a pinacol ester group).

Step 1 is a step of reacting Boronic Acid Compound (II) with a radionuclide selected from ²¹¹At, ²¹⁰At, ¹³¹I, ¹²⁵I, ¹²⁴I, ¹²³I, ⁷⁷Br and ⁷⁶Br in the presence of a reagent selected from an alkali metal iodide, an alkali metal bromide, N-bromosuccinimide, N-chlorosuccinimide, N-iodosuccinimide and hydrogen peroxide, in water to obtain Radiolabeled Compound (I).

Boronic Acid Compound (II) is a novel compound.

In one embodiment, Boronic Acid Compound (II) is preferably Boronic Acid Compound (IIa) shown below. 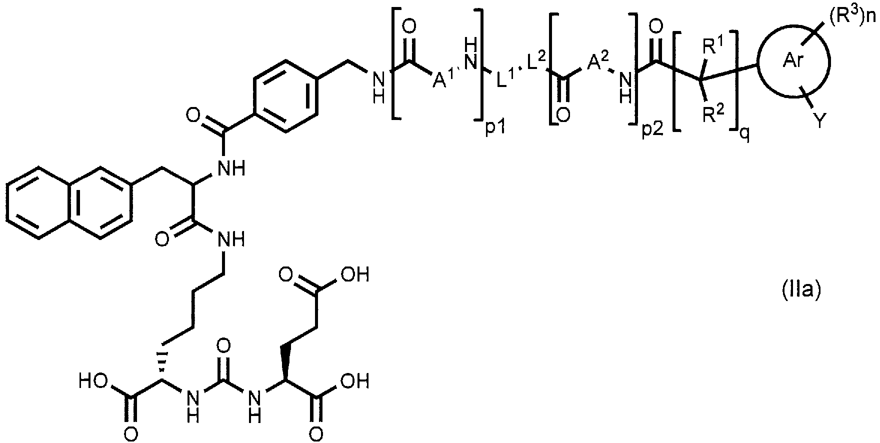 wherein each symbol in the formula is as defined above.

Specific examples of Boronic Acid Compound (II) include the followings.

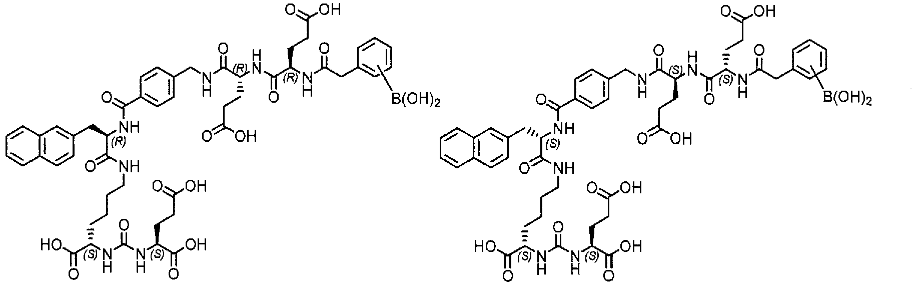

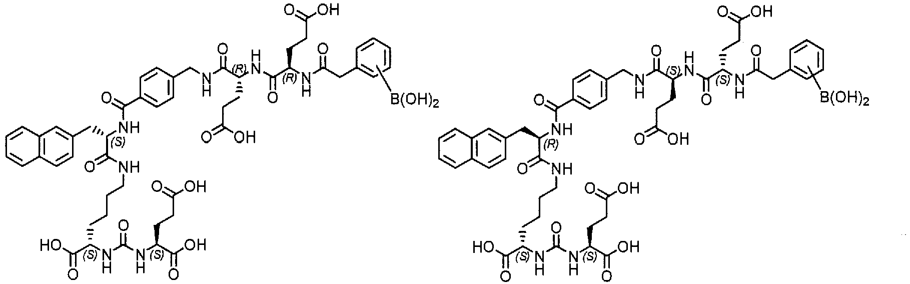

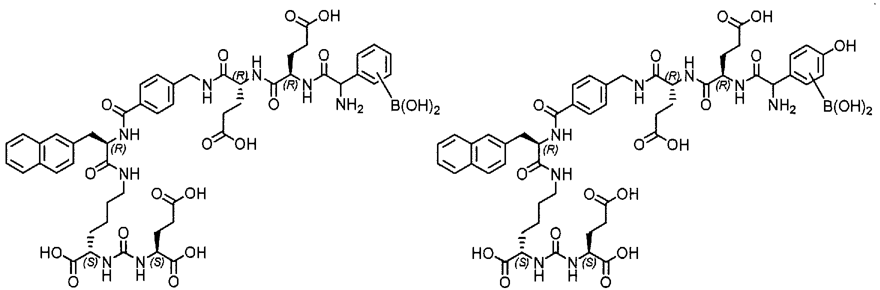

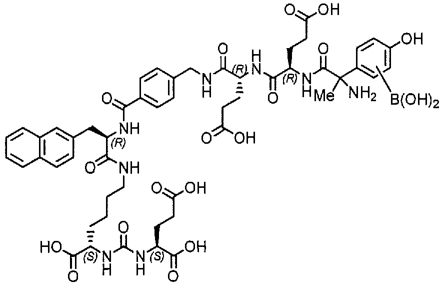

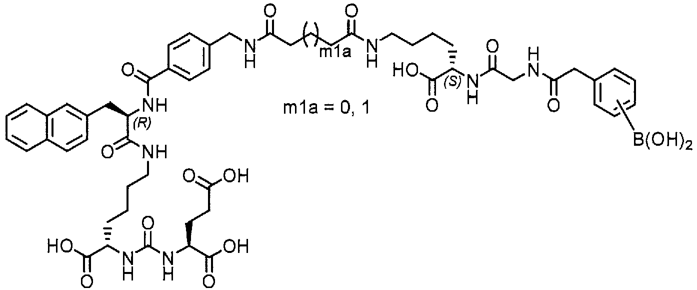

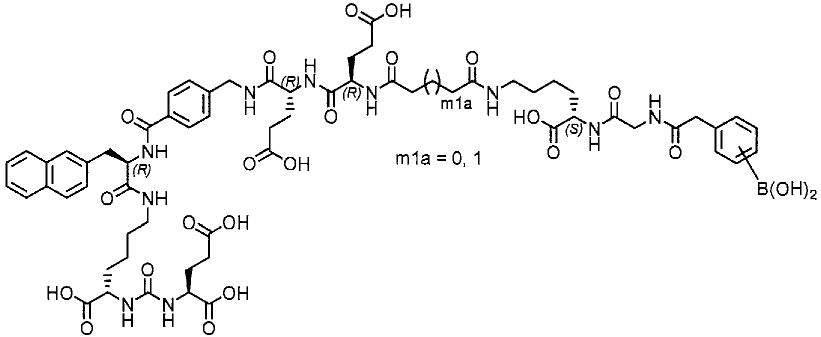

Boronic Acid Compound (II) can be produced by the method described below.

Since the reaction in this step is carried out in water, Boronic Acid Compound (II) may be in a free form or salt form as long as it can be dissolved in water. Alternatively, it may be used in the form of a solution prepared by dissolving in a weakly basic aqueous solution such as an aqueous sodium hydrogen carbonate solution.

Examples of the alkali metal iodide include potassium iodide, sodium iodide and the like. Among them, potassium iodide is preferably used.

Examples of the alkali metal bromide include sodium bromide, potassium bromide and the like.

The preferred combinations of the radionuclide and the above reagent include
(1) a combination in which the radionuclide is ²¹¹At or ²¹⁰At, and the above reagent is selected from potassium iodide, sodium bromide, N-bromosuccinimide, N-chlorosuccinimide, N-iodosuccinimide and hydrogen peroxide;
(2) a combination in which the radionuclide is ¹²³I, ¹²⁴I, ¹²⁵I or ¹³¹I, and the above reagent is selected from N-bromosuccinimide and N-chlorosuccinimide; and
(3) a combination in which the radionuclide is ⁷⁶Br or ⁷⁷Br, and the above reagent is N-chlorosuccinimide.
The above reagent may be used alone or in combination of two or more. The above reagent is used usually in the form of an aqueous solution.

As the preferred embodiment, the radionuclide is ²¹¹At or ¹³¹I, and the reagent is selected from potassium iodide and N-bromosuccinimide.

The more preferred embodiments include
an embodiment in which the radionuclide is ²¹¹At, and the reagent is potassium iodide, and
an embodiment in which the radionuclide is ¹³¹I, and the reagent is N-bromosuccinimide.

The above reagent is used in an amount sufficient to oxidize or reduce the radionuclide, and is used usually in a large excess amount relative to the radionuclide. It is used preferably in a concentration of 0.0001 to 0.2 mol/L, more preferably in a concentration of 0.001 to 0.1 mol/L, in terms of reaction efficiency and economic efficiency.

For the reaction, the radionuclide is used usually in the form of an aqueous solution. If necessary, an alkaline aqueous solution such as sodium hydroxide and buffer solution may be added to the aqueous solution in order to stabilize the radionuclide.

In the case of radionuclide ²¹¹At, first, ²¹¹At is produced by ²⁰⁹Bi(α, 2n) ²¹¹At nuclear reaction resulting from the irradiation of bismuth with helium particles accelerated to 28 MeV by cyclotron. Next, by heating, the target substance ²⁰⁹Bi is melted and the ²¹¹At is vaporized, and the vaporized ²¹¹At is collected in a cooling trap, and dissolved in water to prepare an ²¹¹At stock solution. If necessary, an alkaline solution such as sodium hydroxide and buffer solution may be added thereto for the purpose of stabilizing ²¹¹At.

In the case of radionuclide ²¹⁰At, first, ²¹⁰At is produced by ²⁰⁹Bi(α, 3n)²¹⁰At nuclear reaction resulting from the irradiation of bismuth with helium particles accelerated to 29 MeV or more by cyclotron. Next, by the same procedures as above, an aqueous ²¹⁰At solution is prepared.

In the case of radionuclide ¹²³I, it is available as an aqueous Na¹²³I solution.

In the case of radionuclide ¹²⁴I, first, ¹²⁴I is produced by ¹²⁹Te(p,n)¹²⁴I nuclear reaction resulting from the irradiation of tellurium with proton particles accelerated by cyclotron. Next, the target substance ¹²⁴Te is melted, and the ¹²⁴I is vaporized to prepare an aqueous ¹²⁴I sodium hydroxide solution.

In the case of radionuclide ¹²⁵I, it is available as an aqueous Na¹²⁵I solution.

In the case of radionuclide ¹³¹I, it is available as an aqueous Na¹³¹I solution.

In the case of radionuclide ⁷⁶Br, first, ⁷⁶Br is produced by ⁷⁶Se(p,n)⁷⁶Br nuclear reaction resulting from the irradiation of tellurium with proton particles accelerated by cyclotron. Next, the target substance ⁷⁶Se is melted, and the ⁷⁶Br is vaporized to prepare an aqueous ⁷⁶Br sodium hydroxide solution.

In the case of radionuclide ⁷⁷Br, first, ⁷⁷Br is produced by ⁷⁷Se(p,n)⁷⁷Br nuclear reaction resulting from the irradiation of tellurium with proton particles accelerated by cyclotron. Next, the target substance ⁷⁷Se is melted, and the ⁷⁷Br is vaporized to prepare an aqueous ⁷⁶Br sodium hydroxide solution.

²¹¹At has a half-life of 7.2 hours, ²¹⁰At has a half-life of 8.3 hours, ¹²³I has a half-life of 13.2 hours, and ⁷⁶Br has a half-life of 16 hours. These radionuclides have a short half-life, and therefore, they should be used in the subsequent reaction immediately after the preparation. On the other hand, ¹²⁴I has a half-life of 4.2 days, ¹²⁵I has a half-life of 59.4 days, ¹³¹I has a half-life of 8.04 days, and ⁷⁷Br has a half-life of 57 hours. Although these radionuclides have a relatively long half-life, they are preferably used in the subsequent reaction immediately after the preparation.

Boronic Acid Compound (II) is used usually in a large excess amount relative to the radionuclide, preferably in a concentration of 0.00001 mol/l to 0.5 mol/l, more preferably in a concentration of 0.0001 mol/l to 0.2 mol/l, per 1 Bq to 1,000 GBq of the radionuclide, in terms of reaction efficiency and economic efficiency.

The above reaction is carried out by mixing Boronic Acid Compound (II), the above reagent and the radionuclide, and the mixing order is not particularly limited. The reaction is preferably carried out by adding an aqueous solution of the radionuclide, followed by an aqueous solution of the above reagent to an aqueous solution Boronic Acid Compound (II), or by adding an aqueous solution of the above reagent, followed by an aqueous solution of the radionuclide to an aqueous solution of Boronic Acid Compound (II), more preferably by adding aqueous solution of the radionuclide, followed by an aqueous solution of the above reagent to an aqueous solution of Boronic Acid Compound (II).

The above reaction is carried out in water, i.e., in an organic solvent-free system.

The above reaction is carried out within the range of 0 to 95°C, preferably 10 to 80°C. The reaction time is from 1 minute to 3 hours, preferably from 1 min to 1 hour.

The completion of the reaction is confirmed by the disappearance of the free radionuclide, using thin-layer chromatography (TLC) analysis.

In the production method of the present invention, Radiolabeled Compound (I) can be obtained in a high radiochemical yield of 60% or more, particularly 80% or more, especially 90% or more.

Since the reaction solution after the completion of the reaction contains neither an organic solvent nor a toxic reagent, the reaction solution can be immediately formulated into an injection and the like without isolating Radiolabeled Compound (I).

The reaction of Boronic Acid Compound (II) with a radionuclide is an electrophilic substitution reaction and/or nucleophilic substitution reaction. The introduction site of the radionuclide in Boronic Acid Compound (II) is the benzene ring, which allows the radionuclide to be introduced into the benzene ring well, especially in the case of ²¹¹At or ²¹⁰At.

Radiolabeled Compound (I) may be purified, if necessary, to remove by-products. This purification is preferably carried out by a solid-phase extraction column. As solid-phase extraction columns, those commonly used in the technical field can be used.

Furthermore, after the above purification, ascorbic acid or ascorbate may be added to a final concentration of 0.01% to 10%, preferably 0.1% to 5%. This makes it possible to suppress the decomposition of Radiolabeled Compound (I) and retain it for a long period of time.

Boronic Acid Compound (II) can be produced by the following method comprising Step 2 and Step 3.

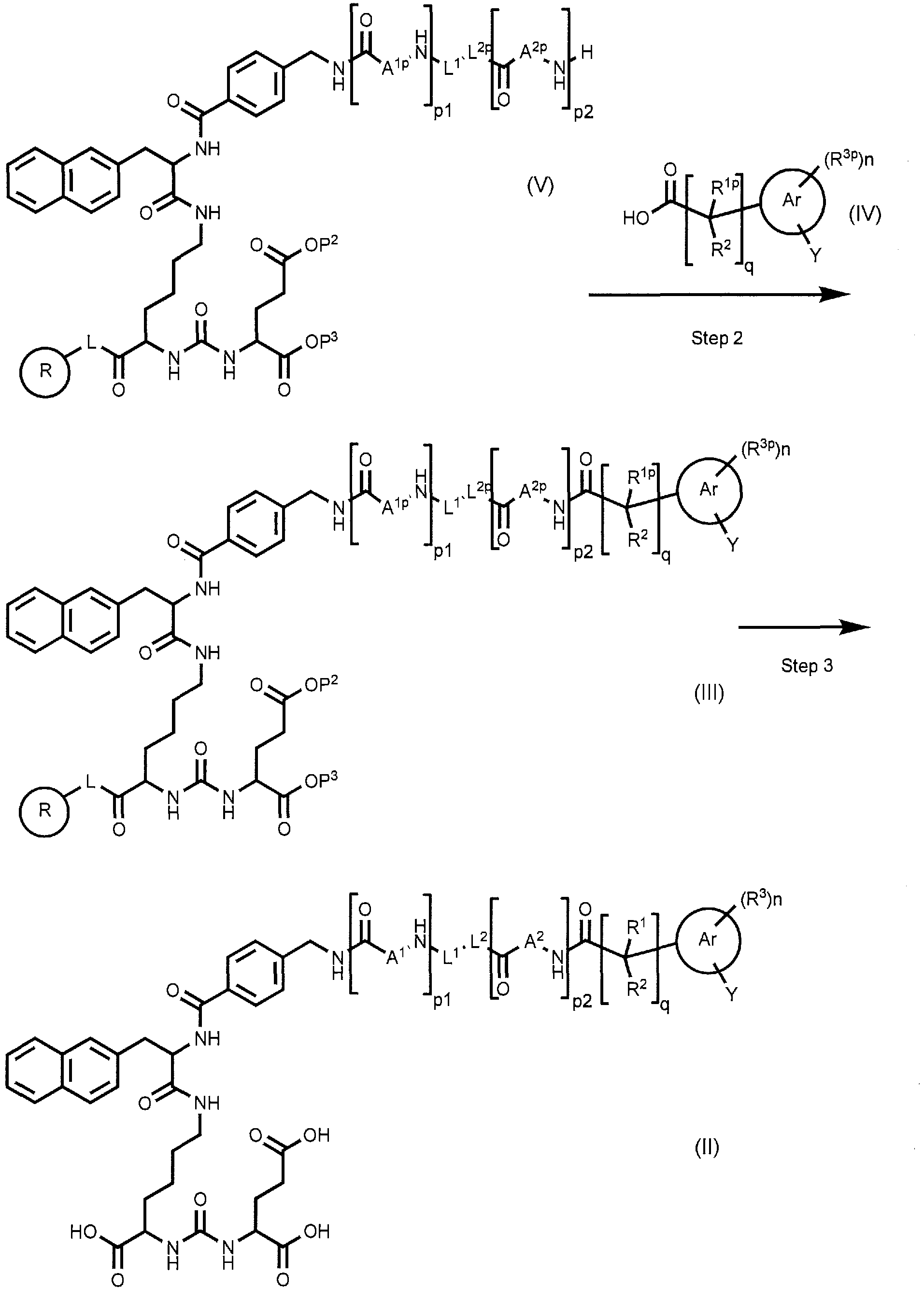

wherein
-CO-A^{1p}-NH- in the number of p1 are each independently a protected amino acid residue;
-CO-A^{2p}-NH- in the number of p2 are each independently a protected amino acid residue;
L^{2p} is a single bond, or -NH- (CH₂)ₘ₂-CH(COOP¹)-NH- wherein P¹ is a carboxy-protecting group, and m2 is as defined above;
R^{1p} in the number of q are each independently a hydrogen atom, a C₁₋₆ alkyl group or a protected amino group;
R^{3p} in the number of n are each independently a C₁₋₆ alkyl group or a protected hydroxy group;
P² and P³ are each independently a carboxy-protecting group, R-L- is a group derived from a resin for solid phase synthesis, and
the other symbols are as defined above.

P¹ is preferably a diphenylmethyl group.

P² and P³ are preferably tert-butyl groups.

Preferable examples of the resin for solid phase synthesis in the group derived from the resin for solid phase synthesis for R-L- include Wang resin and the like.

Step 2 is a step of reacting Compound (V) with Compound (IV) to obtain Compound (III). The reaction is carried out by solid phase synthesis.

Compound (V) is produced by a general solid-phase synthesis method commonly used in peptide synthesis, or by a known synthetic method. After production, Compound (V) is subjected to solid-phase synthesis in Step 2 without deresination.

Compound (IV) may be a commercially available product or can be produced according to a method known per se.

The amount of Compound (IV) to be used is generally 1.5 to 4.0 mol, preferably 3.0 mol or more, per 1 mol of Compound (V) .

The reaction can be carried out in the presence of a condensing agent, or carried out by converting Compound (IV) to the reactive derivative (e.g., an acid chloride), and then reacting the reactive derivative in the presence of a base.

Examples of the condensing agent include (benzotriazol-1-yloxy)tripyrrolidinophosphonium hexafluorophosphate (PyBOP), (benzotriazol-1-yloxy)tris(dimethylamino)phosphonium hexafluorophosphate (BOP), diisopropylcarbodiimide (DIC) and the like. Among them, PyBOP is preferably used.

The amount of the condensing agent to be used is generally 1 mol per 1 mol of Compound (IV).

When the reaction is carried out in the presence of a condensing agent, the reaction may be carried out in the presence of a base. Examples of the base include diisopropylethylamine (DIEA), triethylamine (TEA) and the like. When the condensing agent is PyBOP, DIEA is preferably used.

The amount of the base to be used is generally 1 to 2 mol per 1 mol of Compound (IV).

Examples of the solvent used in the solid-phase synthesis include N-methyl-2-pyrrolidone (NMP), dimethylformamide (DMF) and the like.

The solid-phase synthesis is carried out generally at temperature within the range of 0 to 60°C, preferably 10 to 40°C, generally for 1 to 24 hr, preferably 3 to 12 hr.

Thus-obtained Compound (III) is subjected to Step 3 after washing.

Step 3 is a step of subjecting Compound (III) to deprotection and deresination to obtain Boronic Acid Compound (II) .

The deprotection and deresination is appropriately selected depending on the type of the protecting group and the resin.

For example, when the carboxy-protecting group (P¹, P², P³, etc.) is a tert-butyl group, a diphenylmethyl group or the like, the carboxy-protecting group of the protected amino acid residue (-CO-A^{1p}-NH-, -CO-A^{2p}-NH-) is a tert-butyl group, and R-L- is a group derived from Wang resin, then Boronic Acid Compound (II) can be obtained by treating Compound (III) under an acidic condition.

The treatment under an acidic condition include treatment with an acid such as trifluoroacetic acid.

After the completion of the reaction, the resin for solid-phase synthesis is removed from the reaction mixture, and the resulting mixture was concentrated to obtain Boronic Acid Compound (II). If necessary, Boronic Acid Compound (II) may be purified by HPLC and the like.

The reaction conditions such as solvent and reaction temperature in each step in the production method of the present invention described above are described in detail as representative examples in Examples below, but are not necessarily limited thereto, and those skilled in the art can make appropriate selections based on their general knowledge in organic synthesis.

Thus-produced Radiolabeled Compound (I) binds specifically to prostate-specific membrane antigen (PSMA), and is subsequently taken up and stably accumulated in cells.

Since Radiolabeled Compound (I) targets cells expressing PSMA, Radiolabeled Compound (I) comprising a therapeutically effective radionuclide may be useful in the treatment of tumors or cancers expressing PSMA. Examples of the therapeutically effective radionuclide include ²¹¹At, ²¹⁰At, ¹³¹I, ¹²⁵I and ⁷⁷Br.

Furthermore, since Radiolabeled Compound (I) targets cells expressing PSMA, Radiolabeled Compound (I) comprising an imaging effective radionuclide can image tumors or cancers expressing PSMA, and thus may be useful in the diagnosis of the tumors or cancers. Examples of the imaging effective radionuclide include ²¹¹At, ¹³¹I, ¹²⁴I, ¹²³I, ⁷⁷Br and ⁷⁶Br. Radiolabeled Compound (I) comprising a radionuclide selected from ²¹¹At, ¹³¹I, ¹²⁴I, ¹²³I, ⁷⁷Br and ⁷⁶Br is used for imaging in positron emission tomography (PET) or single photon emission computed tomography (SPECT).

Moreover, Radiolabeled Compound (I) may treat or diagnose tumors or cancers expressing PSMA with few side effects due to accumulation in the kidneys or salivary glands.

The tumor or cancer expressing PSMA include prostate cancer (including pre-metastatic), especially castration-resistant prostate cancer (CRPC), further especially metastatic castration-resistant prostate cancer (mCRPC).

In addition, since all solid tumors essentially express PSMA in neovascular vessels, Radiolabeled Compound (I) can also be used to treat or image almost all solid tumors, including brain tumors, head and neck cancer, lung cancer, mediastinal tumor, breast cancer, malignant tumors of the liver and biliary tract, pancreatic cancer, malignant tumors of the esophagus and gastrointestinal tract such as the stomach and colon, malignant tumors of the kidneys and adrenal glands, malignant tumor of the urinary tract, bladder cancer, sarcomas, malignant melanoma, uterine and ovarian cancers, malignant tumor of the testis, and bone tumors.

The dose of Radiolabeled Compound (I) used for therapeutic or diagnostic purposes is generally determined by the radionuclide used, the patient's weight, age and sex, therapeutic/diagnostic site, and the like. For example, for human subjects, the estimated effective dosage of Radiolabeled Compound (I) with ²¹¹At per dose is approximately 100 MBq to 900 MBq.

Radiolabeled Compound (I) are usually mixed with a pharmaceutically acceptable carrier and used as a pharmaceutical composition. A pharmaceutically acceptable carrier refers to a biocompatible solution with due consideration for sterility, pH, isotonicity, stability, etc, and may include any and all solvents, diluents (including sterile saline, sodium chloride injection, Ringer's solution, dextrose injection, dextrose and sodium chloride injection, lactated Ringer's solution, and other aqueous buffers), dispersants, coatings, antibacterial and antifungal agents, isotonic agents, and the like. Pharmacologically acceptable carriers can also contain stabilizers, preservatives, antioxidants, or other additives known to those skilled in the art.

The dosage form of the pharmaceutical composition is not particularly limited, and it can be prepared as a pharmaceutical composition for oral administration in the form of granules, fine granules, powders, hard capsules, soft capsules, syrups, emulsions, suspensions, liquids and the like; or for parenteral administration such as intravenous administration, intramuscular administration and subcutaneous administration, in the form of injections, drip infusions, transdermal absorptions, transmucosal absorptions, nasal drops, inhalations, suppositories, and the like. These formulations can be prepared according to conventional methods. Preferred are liquid formulations for oral administration or for injection.

Such liquid formulations are prepared by dissolving Radiolabeled Compound (I) in water, but may also be prepared by dissolving Radiolabeled Compound (I) in saline or glucose solution. Buffers or preservatives may be added as necessary. As described above, a reducing agent such as ascorbic acid can also be include. In particular, for the production of injections, the active ingredient is dissolved in distilled water for injection, together with a pH adjuster such as hydrochloric acid, sodium hydroxide, lactose, sodium lactate, sodium monohydrogen phosphate and sodium dihydrogen phosphate, and an isotonic agent such as sodium chloride and glucose, as needed, and then sterilely filtered and filled into an ampule to prepare an injection. Mannitol, dextrin, cyclodextrin, gelatin and the like may also be further added and vacuum lyophilized to prepare an injection that is dissolved immediately before use. Furthermore, lecithin, polysorbate 80, polyoxyethylene hydrogenated castor oil and the like may also be added to the active ingredient and emulsified in water to prepare an emulsion for injection.

The half-life of the radionuclide contained in Radiolabeled Compound (I) is short; it is 7.2 hours for ²¹¹At, 8.3 hours for ²¹⁰At, 8.04 days for ¹³¹I, 59.4 days for ¹²⁵I, 4.2 days for ¹²⁴I, 13.2 hours for ¹²³I, 57 hours for ⁷⁷Br, and 16 hours for ⁷⁶Br. Therefore, it is desirable to prepare the pharmaceutical composition immediately prior to administration to the subject so that it contains the amount of Radiolabeled Compound (I) necessary for administration.

### Examples

The present invention will be explained in detail by the following Examples, which are merely examples and are not intended to limit the present invention and can be modified without departing from the scope of the present invention.

In the following Examples, the radiochemical yield was calculated using the following formula. radiochemical yield (%) = (radioactivity of the desired compound on thin-layer plate/total radioactivity on thin-layer plate) × 100

The abbreviations are as follows.
tBu: tert-butyl
Fmoc: 9-fluorenylmethyloxycarbonyl
Alloc: allyloxycarbonyl
Naph: naphthyl
Ambz: 4-aminomethylbenzoyl
Dpm: diphenylmethyl
Suc: succinyl (-CO-(CH₂)-COOH)
DCM: dichloromethane
DIEA: diisopropylethylamine
NMP: N-methyl-2-pyrrolidone
TCM: trichloromethane
Pd(PPh₃)₄: tetrakis(triphenylphosphine)palladium(0)
DIC: diisopropylcarbodiimide
Oxyma: ethyl cyano(hydroxyimino)acetate
PyBOP: (benzotriazol-1-yloxy)tripyrrolidinophosphonium hexafluorophosphate
TFA: trifluoroacetic acid
TIS: triisopropylsilane
DMSO: dimethyl sulfoxide
DMF: dimethylformamide

### Example 1 Production of Compound 1

Under Ar atmosphere, triphosgene (0.32 g, 1.1 mmol) was dissolved in DCM (80 mL), the solution was cooled to -78°C, and a solution of HCl·H-L-Glu(OtBu)-OtBu (0.80 g, 2.7 mmol) and DIEA (4.6 mL, 27 mmol) in DCM (24 mL) was added dropwise thereto. After 30 min, the mixture was warmed to room temperature, H-L-Lys(Alloc)-Wang resin prepared by deprotecting Fmoc-L-Lys(Alloc)-Wang resin (0.54 mmol) with 20% piperidine/NMP was added thereto, and the mixture was stirred for 1.5 hr. The resin was washed, and to the resin in TCM (10 mL) were added Pd(PPh₃)₄ (94 mg, 0.08 mmol) and phenylsilane (3.3 mL, 27 mmol), and the mixture was stirred under Ar atmosphere for 1 hr. After the resin was washed, the procedures of condensation with sequential Fmoc-D-Ala(2-Naph)-OH, Fmoc-(4)Ambz-OH, Fmoc-D-Glu(OtBu) and Fmoc-D-Glu(OtBu) by DIC-Oxyma and deprotection with 20% piperidine/NMP were repeated to elongate peptide chain. Then, 0.22 mmol (40%) of the obtained resin was taken, and to the resin in NMP (15 mL) were added 4-(carboxymethyl)phenylboronic acid (0.12 g, 0.68 mmol), PyBOP (0.35 g, 0.68 mmol) and DIEA (0.13 mL, 0.79 mmol), the mixture was stirred for 1.5 hr, and the resin was washed to obtain a protected peptide resin. To the obtained protected peptide resin was added trifluoroacetic acid cocktail (30 mL, TFA/TIS/H₂O=95/2.5/2.5), and the mixture was stirred at room temperature for 1 hr to deprotect and deresinate the protected peptide resin. The resin was removed by filtration, the trifluoroacetic acid solution was concentrated, and the crude peptide was solidified with diethyl ether and collected by filtration. Finally, the obtained crude peptide was dissolved in 30% aqueous DMSO solution, and purified by reverse-phase HPLC. The fraction containing the target product was lyophilized to obtain Compound 1 as a lyophilized powder (yield: 54 mg, purity 99% or more (HPLC method), molecular weight: observed value 1070.4[M+H]⁺, 1052.4[M-H₂O+H]⁺, theoretical value 1070.4[M+H]).

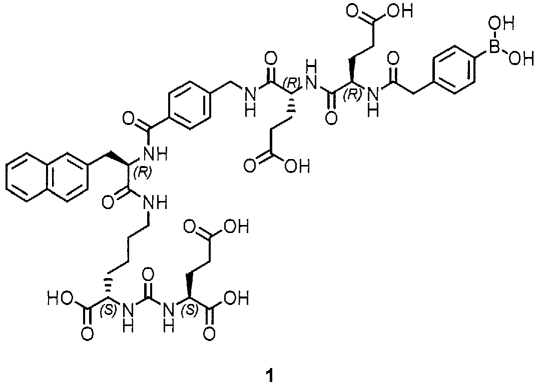

### Example 2 Production of Compound 2

Compound 2 was synthesized by the same process as in Example 1, except that Fmoc-D-Ala(2-Naph)-OH and Fmoc-D-Glu(OtBu) were changed to Fmoc-L-Ala(2-Naph)-OH and Fmoc-L-Glu(OtBu) (yield: 204 mg, purity 99% or more (HPLC method), molecular weight: observed value 1070.3[M+H]⁺, 1052.4[M-H₂O+H]⁺, theoretical value 1070.4[M+H]).

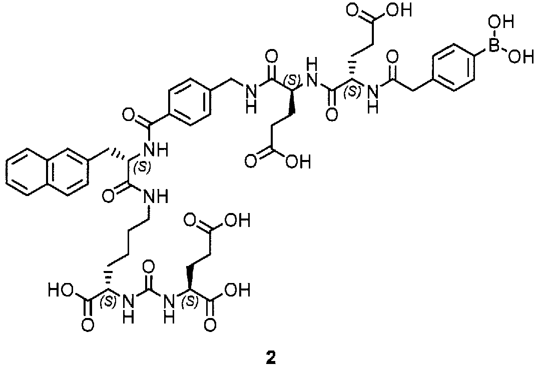

### Example 3 Production of Compound 3

The powder of Compound 1 obtained in Example 1 was dissolved in 7% aqueous sodium hydrogencarbonate solution to 0.1 mg/mL. To the solution (10 µL) were added ²¹¹At aqueous solution (20 µL, 20 MBq) and 0.1 mol/L aqueous potassium iodide solution (30 µL), and the reaction was carried out at 80°C for 1 hr. The reaction solution was injected into a solid-phase extraction cartridge (Oasis HLB, Waters), the cartridge was washed with water (1 mL), and 30% aqueous ethanol solution (0.5 mL) was injected into the cartridge to collect the eluate. The above reaction solution and eluate were analyzed by thin-layer chromatograph method (TLC). Silica gel 60 (Merck) was used as a thin plate and developed with acetonitrile/water mixture (2/1). The radioactivity on the thin plate after development was exposed to an imaging plate (GE Healthcare) and analyzed by a bioimaging analyzer (BAS7000, GE Healthcare). The results were shown in Figure 1. From the results of TLC analysis of the reaction solution, the radiochemical yield of Compound 3 was 58.0%, and the radiochemical purity of the eluate was 98.6%.

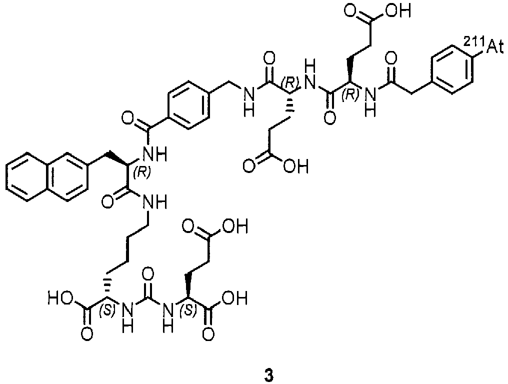

### Example 4 Production of Compound 4

The powder of Compound 2 obtained in Example 2 was dissolved in 7% aqueous sodium hydrogencarbonate solution to 0.1 mg/mL. To the solution (10 µL) were added ²¹¹At aqueous solution (20 µL, 20 MBq) and 0.1 mol/L aqueous potassium iodide solution (30 µL), and the reaction was carried out at 80°C for 1 hr. The reaction solution was injected into a solid-phase extraction cartridge (Oasis HLB, Waters), the cartridge was washed with water (1 mL), and 30% aqueous ethanol solution (0.5 mL) was injected into the cartridge to collect the eluate. The above reaction solution and eluate were analyzed by thin-layer chromatograph method (TLC). Silica gel 60 (Merck) was used as a thin plate and developed with acetonitrile/water mixture (2/1). The radioactivity on the thin plate after development was exposed to an imaging plate (GE Healthcare) and analyzed by a bioimaging analyzer (BAS7000, GE Healthcare). The results were shown in Figure 2. From the results of TLC analysis of the reaction solution, the radiochemical yield of Compound 4 was 72.6%, and the radiochemical purity of the eluate was 99.9%.

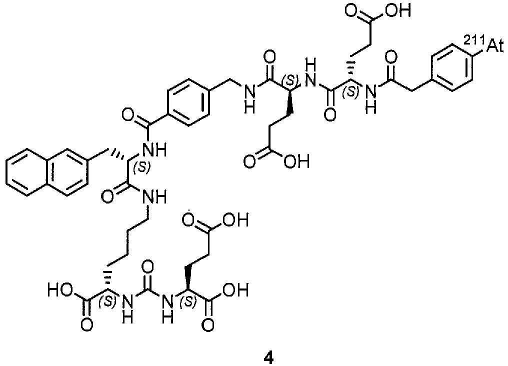

### Reference Example 1 Synthesis of Fmoc-L-Lys(Suc)-ODpm

HCl·Fmoc-L-Lys-ODpm (2.5 g, 4.4 mmol) was dissolved in DMF (20 mL), succinic anhydride (0.48 g, 4.8 mmol) and DIEA (0.82 mL, 4.8 mmol) were added thereto, and the mixture was stirred at room temperature for 1.5 hr. To the reaction solution was added ethyl acetate, and the mixture was washed with 0.2 N hydrochloric acid water and brine, and dried over magnesium sulfate, and the solvent was evaporated under reduced pressure to give Fmoc-L-Lys(Suc)-ODpm (2.1 g, 89%).

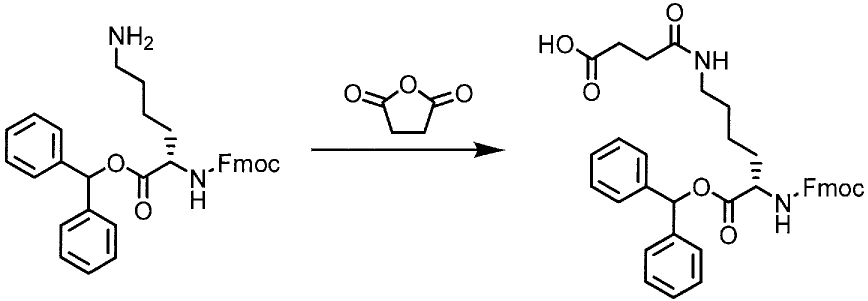

### Example 5 Production of Compound 5

Under Ar atmosphere, triphosgene (0.42 g, 1.4 mmol) was dissolved in dichloromethane (90 mL), the mixture was cooled to -78°C, and a solution of HCl·H-L-Glu(OtBu)-OtBu (1.0 g, 3.5 mmol) and DIEA (6.0 mL, 35 mmol) in dichloromethane (30 mL) was added dropwise thereto. After 30 min, the mixture was warmed to room temperature, H-L-Lys(Alloc)-Wang resin prepared by deprotecting Fmoc-L-Lys(Alloc)-Wang resin (0.7 mmol) with 20% piperidine/NMP was added thereto, and the mixture was stirred for 2 hr. The resin was washed, and to the resin in chloroform (10 mL) were added Pd(PPh₃)₄ (0.12 g, 0.11 mmol) and phenylsilane (4.3 mL, 35 mmol), and the mixture was stirred under Ar atmosphere for 1 hr. After the resin was washed, the procedures of condensation with sequential Fmoc-D-Ala(2-Naph)-OH, Fmoc-(4)Ambz-OH, Fmoc-L-Lys(Suc)-ODpm (obtained Reference Example 1) and Fmoc-Gly-OH by DIC-Oxyma and deprotection with 20% piperidine/NMP were repeated to elongate peptide chain. Then, to the resin in NMP (15 mL) were added 4-(carboxymethyl)phenylboronic acid pinacol ester (0.55 g, 2.1 mmol), PyBOP (1.1 g, 2.1 mmol) and DIEA (0.42 mL, 2.5 mmol), the mixture was stirred for 1 hr, and the resin was washed to obtain a protected peptide resin. To the obtained protected peptide resin was added trifluoroacetic acid cocktail (60 mL, TFA/TIS/H₂O=95/2.5/2.5), and the mixture was stirred at room temperature for 1 hr to deprotect and deresinate the protected peptide resin. The resin was removed by filtration, the solvent of the filtrate was evaporated under reduced pressure, and the residue was solidified and washed with diethyl ether. The obtained solid was dissolved in 50% aqueous acetic acid (10 mL), and the solution was stirred at room temperature for 40 min, and purified by reverse-phase HPLC. The fraction containing the target product was lyophilized to give Compound 5 as a lyophilized powder (yield 0.20 g, yield 26%, purity 98% or more (HPLC method), molecular weight: observed value 1097.5[M+H]⁺, 1079.5[M-H₂O+H]⁺, theoretical value 1097.5[M+H]).

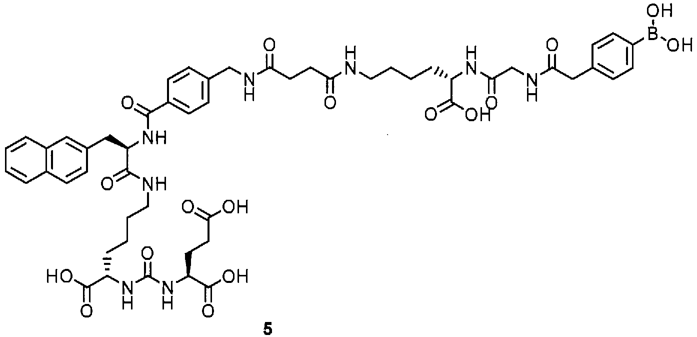

### Example 6 Production of Compound 6

Compound 6 was synthesized by the same process as in Example 5, except that the peptide chain to be elongated was changed from Fmoc-D-Ala(2-Naph)-OH, Fmoc-(4)Ambz-OH, Fmoc-L-Lys(Suc)-ODpm and Fmoc-Gly-OH to Fmoc-D-Ala(2-Naph)-OH, Fmoc-(4)Ambz-OH, Fmoc-D-Glu(OtBu)-OH, Fmoc-D-Glu(OtBu)-OH, Fmoc-L-Lys(Suc)-ODpm and Fmoc-Gly-OH (yield 0.28 g, yield 29%, purity 98% or more (HPLC method), molecular weight: observed value 1353.5[M-H]⁻, 1335.5[M-H₂O-H]⁻, theoretical value 1353.5[M-H])

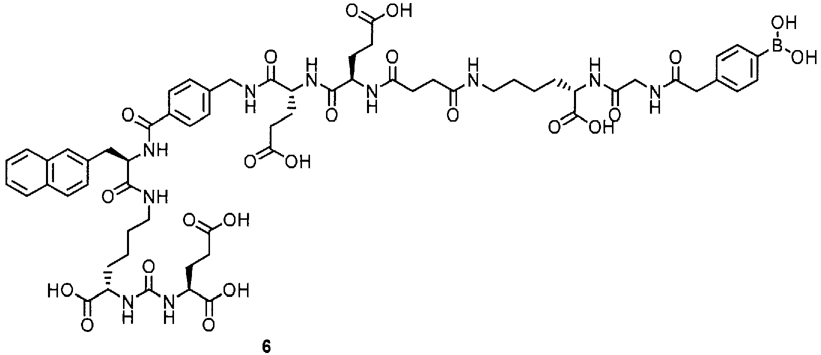

### Example 7 Production of Compound 7

The powder of Compound 5 obtained in Example 5 was dissolved in 7% aqueous sodium hydrogencarbonate solution to 0.1 mg/mL. To the solution (20 µL) were added 7% aqueous sodium hydrogencarbonate solution (50 µL), ²¹¹At aqueous solution (16 µL, 38.5 MBq) and 0.1 mol/L aqueous potassium iodide solution (40 µL), and the reaction was carried out at 80°C for 45 min. The reaction solution was injected into a solid-phase extraction cartridge (Oasis HLB, Waters), the cartridge was washed with water (1 mL), and 20% ethanol aqueous solution (0.5 mL) and 30% ethanol aqueous solution were injected successively into the cartridge to collect the eluate. The above reaction solution and 30% ethanol eluate were analyzed by thin-layer chromatograph method (TLC). Silica gel 60 (Merck) was used as a thin plate and developed with acetonitrile/water mixture (2/1). The radioactivity on the thin plate after development was exposed to an imaging plate (GE Healthcare) and analyzed by a bioimaging analyzer (BAS7000, GE Healthcare). The results were shown in Figure 3. From the results of TLC analysis of the reaction solution, the radiochemical yield of Compound 7 was 67.4%, and the radiochemical purity of the eluate was 96.3%.

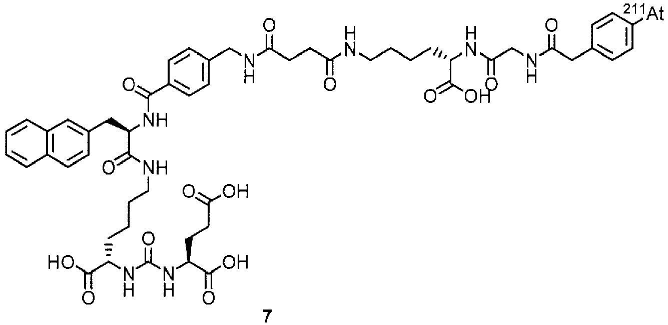

### Example 8 Production of Compound 8

The powder of Compound 6 obtained in Example 6 was dissolved in 7% aqueous sodium hydrogencarbonate solution to 0.1 mg/mL. To the solution (20 µL) were added 7% aqueous sodium hydrogencarbonate solution (40 µL), ²¹¹At aqueous solution (22 µL, 20 MBq) and 0.1 mol/L aqueous potassium iodide solution (40 µL), and the reaction was carried out at 80°C for 45 min. The reaction solution was injected into a solid-phase extraction cartridge (Oasis HLB, Waters), the cartridge was washed with water (1 mL), and 20% ethanol aqueous solution (0.5 mL) was injected into the cartridge to collect the eluate. The above reaction solution and eluate were analyzed by thin-layer chromatograph method (TLC). Silica gel 60 (Merck) was used as a thin plate and developed with acetonitrile/water mixture (2/1). The radioactivity on the thin plate after development was exposed to an imaging plate (GE Healthcare) and analyzed by a bioimaging analyzer (BAS7000, GE Healthcare). The results were shown in Figure 4. From the results of TLC analysis of the reaction solution, the radiochemical yield of Compound 8 was 61.5%, and the radiochemical purity of the eluate was 97.7%.

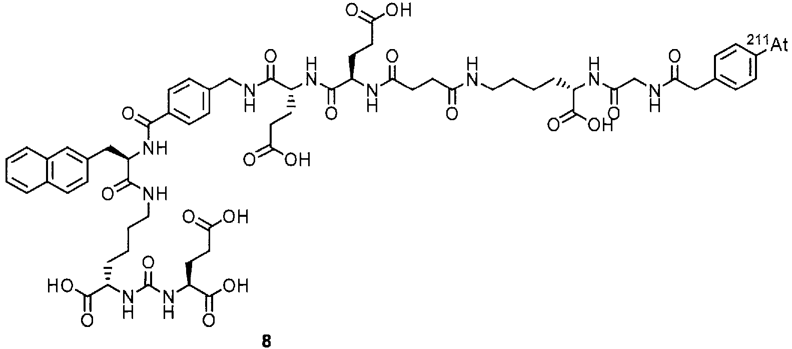

### Experimental Example 1 Imaging of human prostate cancer-transplanted mice with Compound 3

SCID mice (9 weeks old, male, n=5) were subcutaneously transplanted with human prostate cancer cells (LNCaP, 0.5×10⁷ cells/mouse), and then kept for one month. The mice were divided into Compound 3 administration group (n=3) and control group (n=2). For the Compound 3 administration group, Compound 3 (0.43±0.01 MBq) was administered via the tail vein. For the control group, physiological saline was administered. The mice in the Compound 3 administration group (n=3) were anesthetized with isoflurane inhalation at 3 and 24 hours after administration, and the planar images were taken using a SPECT camera (E-cam, Siemens) (matrix size: 256x256, pixel size 1.2x1.2mm, collimator: LEAP, energy window: 79keV±20%, image acquisition time 10 min or 20 min). The results were shown in Figure 5. It was found that Compound 3 accumulated at the tumor-transplanted site (arrow) 3 hours after administration, and the accumulation continued even 24 hours after administration. Compound 3 also showed physiological accumulation in the kidneys, but no nonspecific accumulation in other organs was observed.

### Experimental Example 2 Therapeutic test in human prostate cancer-transplanted mice with Compound 3

The mice in the Compound 3 administration group (0.5 MBq, n=3) and control group (CTL, n=2) of Experimental Example 1 were kept for 3 weeks thereafter, and changes in tumor size were measured. The tumor size was standardized by size at the time of drug administration, and the relative ratio (fold change) to subsequent tumor size was calculated. The results were shown in Figure 6. The tumor size in the control group increased over time, and it increased about seven times after three weeks. On the other hand, the tumor size in the drug-administered group showed a tendency to shrink immediately after administration, and it decreased to 0.27 times after three weeks. The mice were dissected and blood biochemical tests were performed. There were no significant differences in renal function marker values between the control group and the drug-administered group. As a result of histopathological examination, no particular abnormal findings were observed in the kidneys, salivary glands and thyroid glands of mice in the drug-administered group.

The above results showed that Compound 3 is useful as a therapeutic agent for prostate cancer.

### Experimental Example 3 Biodistribution test in human prostate cancer-transplanted mice

SCID mice (9 weeks old, male, n=5) were subcutaneously transplanted with human prostate cancer cells (LNCaP, 0.5×10⁷ cells/mouse), and then kept for one month. Compound 3, 4, 7 or 8 (approximately 0.1 MBq) was administered to the tumor-transplanted mice or normal mice, and the mice were dissection under isoflurane inhalation anesthesia at 3 and 24 hours after administration to collect various organs including blood, urine and tumors. The radioactivity distribution (%ID) and radioactivity distribution per 1 g of organ weight (%ID/g) were measured for each organ. The results were shown in Figures 7 to 11. Compounds 3 and 7 were both significantly accumulated in the tumors. In addition, all Compounds 3, 4, 7 and 8 were observed to accumulate physiologically in the kidney.

### Experimental Example 4 Therapeutic test in human prostate cancer-transplanted mice

SCID mice (9 weeks old, male, n=5) were subcutaneously transplanted with human prostate cancer cells (LNCaP, 0.5×10⁷ cells/mouse), and then kept for one month. The tumor-transplanted mice were divided into Compound 3, 7 or 8 administration group (0.4MBq or 1MBq, n=3) and control group (CTL, n=2 or 3), and kept for 3 weeks thereafter, and changes in tumor size and body weight were measured. The results were shown in Figures 12 to 14. The tumor size in the control group increased over time, whereas tumor growth inhibition was observed in all Compound 3, 7 and 8 administration groups.

### Industrial Applicability

According to the present invention, a radiolabeled compound that binds specifically to PSMA, is effective in the treatment and diagnosis of tumors or cancers expressing PSMA, for example, the treatment and diagnosis of prostate cancer, especially castration-resistant prostate cancer (CRPC), further especially metastatic castration-resistant prostate cancer (mCRPC), and does not exhibit side effects due to accumulation in the kidney or salivary glands, can be provided.

This application is based on patent application No. 2021-125774 filed on July 30, 2021 in Japan, the contents of which are encompassed in full herein.

## Claims

1. A radiolabeled compound represented by Formula (I) or a pharmaceutically acceptable salt thereof: 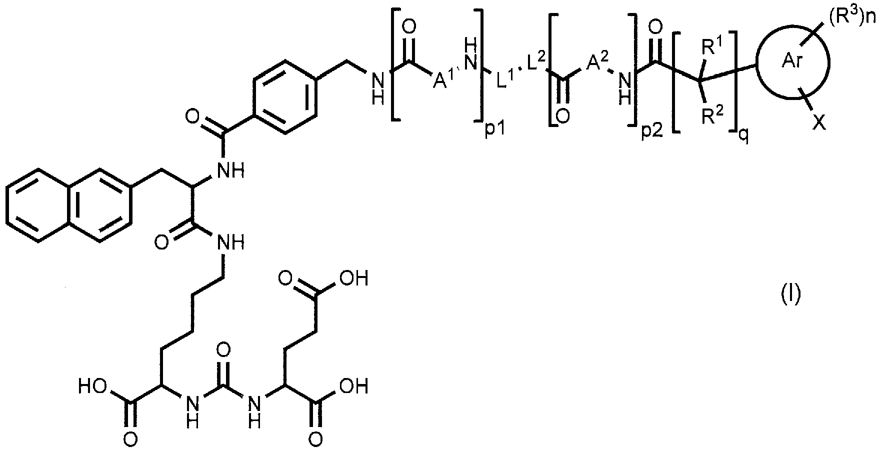 wherein
-CO-A¹-NH- in the number of p1 are each independently an amino acid residue;
-CO-A²-NH- in the number of p2 are each independently an amino acid residue;
L¹ is a single bond, or -CO-(CH₂)ₘ₁-CO- wherein m1 is an integer of 1 to 6;
L² is a single bond, or -NH-(CH₂)ₘ₂-CH(COOH)-NH- wherein m2 is an integer of 1 to 6;
Ar is a C₆₋₁₄ aryl group;
R¹ in the number of q are each independently a hydrogen atom, a C₁₋₆ alkyl group or an amino group;
R² in the number of q are each independently a hydrogen atom or a C₁₋₆ alkyl group;
R³ in the number of n are each independently a C₁₋₆ alkyl group or a hydroxy group;
X is a radionuclide selected from ²¹¹At, ²¹⁰At, ¹³¹I, ¹²⁵I, ¹²⁴I, ¹²³I, ⁷⁷Br and ⁷⁶Br;
p1 is an integer of 0 to 3;
p2 is an integer of 0 to 3;
q is an integer of 0 to 3; and
n is an integer of 0 to 3.

2. The compound according to Claim 1 or a pharmaceutically acceptable salt thereof, wherein L¹ and L² are both single bonds.

3. The compound according to Claim 1 or a pharmaceutically acceptable salt thereof, wherein L¹ is -CO-(CH₂)ₘ₁-CO- wherein m1 is as defined in Claim 1, and L² is -NH-(CH₂)ₘ₂-CH(COOH)-NH-wherein m2 is as defined in Claim 1.

4. The compound according to any one of Claim 1 to 3 or a pharmaceutically acceptable salt thereof, wherein at least one of -CO-A¹-NH- in the number of p1 is a glutamic acid residue.

5. The compound according to any one of Claim 1 to 3 or a pharmaceutically acceptable salt thereof, wherein p1 is an integer of 0 to 2.

6. The compound according to any one of Claim 1 to 3 or a pharmaceutically acceptable salt thereof, wherein at least one of -CO-A²-NH- in the number of p2 is a glycine acid residue.

7. The compound according to any one of Claim 1 to 3 or a pharmaceutically acceptable salt thereof, wherein p2 is 0 or 1.

8. The compound according to any one of Claim 1 to 3 or a pharmaceutically acceptable salt thereof, wherein Ar is a phenyl group.

9. The compound according to any one of Claim 1 to 3 or a pharmaceutically acceptable salt thereof, wherein R¹ and R² are hydrogen atoms.

10. The compound according to any one of Claim 1 to 3 or a pharmaceutically acceptable salt thereof, wherein q is an integer of 1 to 3.

11. A pharmaceutical composition comprising a compound as defined in any one of Claim 1 to 3 or a pharmaceutically acceptable salt thereof, and pharmaceutically acceptable carrier.

12. A therapeutic agent for a tumor or cancer expressing prostate-specific membrane antigen (PSMA), comprising a compound as defined in any one of Claim 1 to 3 or a pharmaceutically acceptable salt thereof.

13. The agent according to Claim 12, wherein the tumor or cancer expressing PSMA is prostate cancer.

14. A diagnostic agent for a tumor or cancer expressing prostate-specific membrane antigen (PSMA), comprising a compound as defined in any one of Claim 1 to 3 or a pharmaceutically acceptable salt thereof.

15. The agent according to Claim 14, wherein the tumor or cancer expressing PSMA is prostate cancer.

16. A compound represented by Formula (II) or a salt thereof: 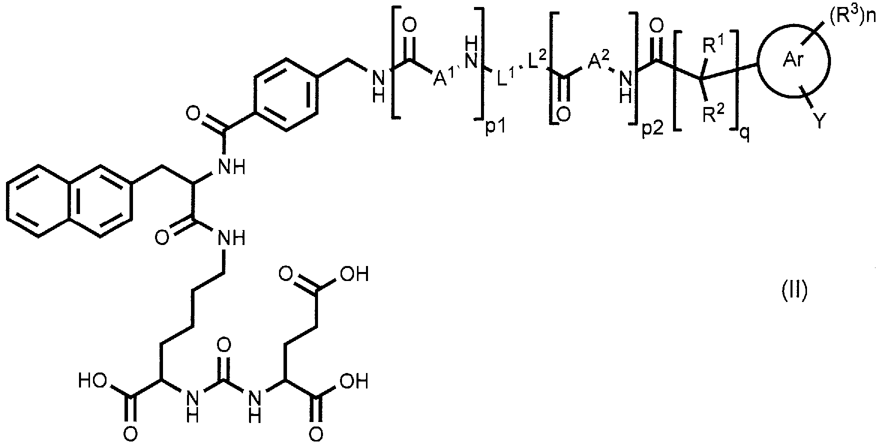 wherein
-CO-A¹-NH- in the number of p1 are each independently an amino acid residue;
-CO-A²-NH- in the number of p2 are each independently an amino acid residue;
L¹ is a single bond, or -CO-(CH₂)ₘ₁-CO- wherein m1 is an integer of 1 to 6;
L² is a single bond, or -NH-(CH₂)ₘ₂-CH(COOH)-NH- wherein m2 is an integer of 1 to 6;
Ar is a C₆₋₁₄ aryl group;
R¹ in the number of q are each independently a hydrogen atom, a C₁₋₆ alkyl group or an amino group;
R² in the number of q are each independently a hydrogen atom or a C₁₋₆ alkyl group;
R³ in the number of n are each independently a C₁₋₆ alkyl group or a hydroxy group;
Y is a boryl group (-B(OH)₂) or its ester group;
p1 is an integer of 0 to 3;
p2 is an integer of 0 to 3;
q is an integer of 0 to 3; and
n is an integer of 0 to 3.

17. The compound according to Claim 16 or a salt thereof, wherein Y is a boryl group (-B(OH)₂) or a 4,4,5,5-tetramethyl-1,3,2-dioxaboran-2-yl group.

18. A method for producing a radiolabeled compound represented by Formula (I) of a pharmaceutically acceptable salt thereof, comprising the following step; 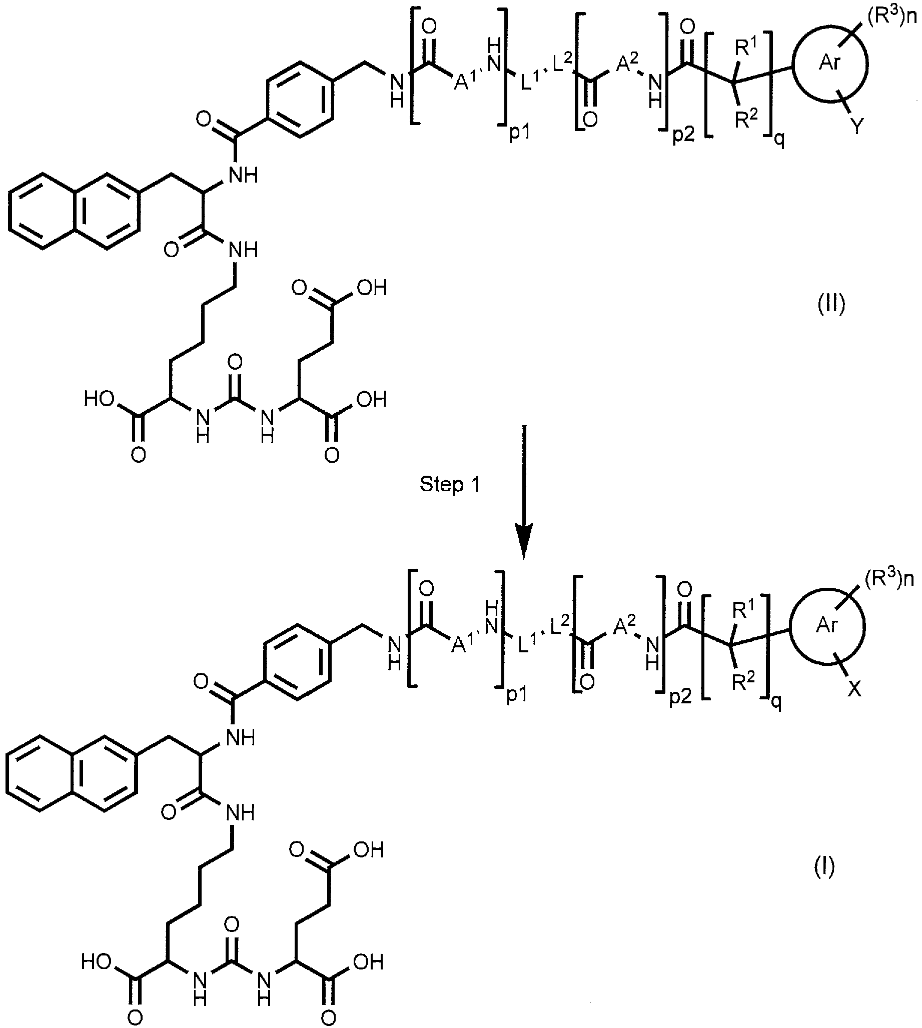 wherein
-CO-A¹-NH- in the number of p1 are each independently an amino acid residue;
-CO-A²-NH- in the number of p2 are each independently an amino acid residue;
L¹ is a single bond, or -CO-(CH₂)ₘ₁-CO- wherein m1 is an integer of 1 to 6;
L² is a single bond, or -NH-(CH₂)ₘ₂-CH(COOH)-NH- wherein m2 is an integer of 1 to 6;
Ar is a C₆₋₁₄ aryl group;
R¹ in the number of q are each independently a hydrogen atom, a C₁₋₆ alkyl group or an amino group;
R² in the number of q are each independently a hydrogen atom or a C₁₋₆ alkyl group;
R³ in the number of n are each independently a C₁₋₆ alkyl group or a hydroxy group;
X is a radionuclide selected from ²¹¹At, ²¹⁰At, ¹³¹I, ¹²⁵I, ¹²⁴I, ¹²³I, ⁷⁷Br and ⁷⁶Br;
Y is a boryl group (-B(OH)₂) or its ester group;
p1 is an integer of 0 to 3;
p2 is an integer of 0 to 3;
q is an integer of 0 to 3; and
n is an integer of 0 to 3,
Step 1: a step of reacting a compound represented by Formula (II) or a salt thereof with a radionuclide selected from ²¹¹At, ²¹⁰At, ¹³¹I, ¹²⁵I, ¹²⁴I, ¹²³I, ⁷⁷Br and ⁷⁶Br in the presence of a reagent selected from an alkali metal iodide, an alkali metal bromide, N-bromosuccinimide, N-chlorosuccinimide, N-iodosuccinimide and hydrogen peroxide, in water, to obtain a radiolabeled compound represented by Formula (I) or a pharmaceutically acceptable salt thereof.
